# EUROPÄISCHE PATENTSCHRIFT

(11) **EP 3 167 817 B1**
(45) Veröffentlichungstag und Bekanntmachung des Hinweises auf die Patenterteilung: **04.08.2021**
(21) Anmeldenummer: 16187605.7
(22) Anmeldetag: 07.09.2016
(51) Int. Cl.: A61B 17/00, A61B 34/30, A61B 34/00, A61B 17/072

(54) **MANIPULATIONSSYSTEM SOWIE HANDHABUNGSVORRICHTUNG FÜR CHIRURGISCHE INSTRUMENTE**
MANIPULATION SYSTEM AND HANDLING DEVICE FOR SURGICAL INSTRUMENTS
SYSTEME ET DISPOSITIF DE MANIPULATION POUR DES INSTRUMENTS CHIRURGICAUX

(30) Priorität: 15.09.2015 DE 102015115559
(43) Veröffentlichungstag der Anmeldung: 17.05.2017
(73) Patentinhaber: Karl Storz SE & Co. KG, 78532 Tuttlingen (DE)
(72) Erfinder: Schlegel, Sebastian, 10825 Berlin (DE); Blase, Bastian, 10439 Berlin (DE)
(74) Vertreter: Witte, Weller & Partner Patentanwälte mbB

(56) Entgegenhaltungen:
- EP-A2- 2 767 243
- EP-A2- 2 792 313
- EP-A2- 2 889 009
- DE-A1-102012 008 537
- US-A1- 2011 277 775

## Beschreibung

Die vorliegende Offenbarung betrifft eine Handhabungsvorrichtung zur Aufnahme und Handhabung von Instrumenten für minimal-invasive Eingriffe, mit einem Gestell und zumindest einem Instrumententräger mit einem Aufnahmeabschnitt zur Aufnahme eines Instrumentenarms. Die Offenbarung betrifft ferner ein Manipulationssystem für minimal-invasive chirurgische Operationen und sonstige Eingriffe, insbesondere ein Telemanipulationssystem für minimal-invasive Single-Port-Operationen, mit einer Ansteuerungsplattform, die eine derartige Handhabungsvorrichtung aufweist.

Allgemein betrifft die vorliegende Offenbarung das Gebiet der minimal-invasiven Chirurgie sowie das Gebiet robotischer Operationssysteme bzw. das Gebiet robotischer Teleoperationssysteme. Bei minimal-invasiven Eingriffen nutzen Chirurgen üblicherweise natürliche Körperöffnungen oder künstlich geschaffene Körperöffnungen, um verschiedene Instrumente ins Innere des Körpers einer zu behandelnden Person bzw. eines zu behandelnden Tieres einzuführen. Minimal-invasive Eingriffe können Eingriffe therapeutischer Art, Eingriffe diagnostischer Art sowie Kombinationen davon umfassen. Allgemein wird angestrebt, nur möglichst kleine Zugangsöffnungen zum Körper zu verwenden, um die Belastung für den Patienten möglichst zu minimieren. Häufig erlauben Instrumente für minimal-invasive Eingriffe nur begrenzte Bewegungen bzw. Operationen im Innern des Körpers.

Aus der EP 2 792 313 A2 ist ein chirurgisches Befestigungsinstrument bekannt, umfassend eine erste Backe, eine zweite Backe mit einer Befestigungskartusche und ein Verschlusssystem. Das Schließsystem umfasst ein Verschlusselement, das konfiguriert ist, um in die erste Backe einzugreifen und die erste Backe in Richtung der zweiten Backe zu bewegen. Ein Drehgelenk verbindet die erste Backe schwenkbar mit der zweiten Backe. Das Verschlusselement greift in den ersten Backen an einer Stelle ein, die in Bezug auf den Drehpunkt distal liegt. Das Verschlusselement und die erste Backe beinhalten eine Nockenstift-Nockenschlitzanordnung, die die Hebelwirkung oder den mechanischen Vorteil erhöht, den das Verschlusselement auf die erste Backe ausübt, wenn die erste Backe aus einer geöffneten Position in eine geschlossene Position bewegt wird. Das chirurgische Instrument umfasst ferner einen ersten Motor, der konfiguriert ist, um das Verschlusssystem zu betreiben, und einen zweiten Motor, der konfiguriert ist, um ein Zündsystem zu betreiben, das konfiguriert ist, um Befestigungselemente aus der Befestigungskartusche auszuwerfen.

Aus der EP 2 767 243 A2 ist eine elektromechanische chirurgische Vorrichtung bekannt, umfassend einen End-Effektor, der eine davon vorstehende Eingangsantriebsachse umfasst; und eine Wellenanordnung. Die Wellenanordnung beinhaltet eine drehbare Antriebswelle; ein proximales Halsgehäuse, das an einem distalen Ende eines Außenrohrs abgestützt ist; ein distales Halsgehäuse (236), das schwenkbar mit dem proximalen Halsgehäuse verbunden ist; einen Drehbolzen, der das proximale Halsgehäuse und das distale Halsgehäuse verbindet; und einen Zahnradstrang, der in dem proximalen Halsgehäuse, auf dem Drehbolzen und in dem distalen Halsgehäuse abgestützt ist. Der Zahnradstrang beinhaltet ein proximales Zahnrad, ein Zwischenzahnrad, ein distales Zahnrad und ein Paar Abtriebszahnräder, wobei jedes Abtriebszahnrad eine Kupplungsbuchse definiert, die jeweils konfiguriert ist, um die Antriebsachse des Endeffektors selektiv aufzunehmen.

Aus der EP 0 836 425 A1 ist chirurgischer Instrumentenmanipulator bekannt, mit einer fixierbaren Trägerbasis, einem Instrumentenhalter, der beweglich an der Trägerbasis angebracht und angepasst ist, um ein chirurgisches Instrument lösbar zu halten, einer Antriebsanordnung, und mit einer Kopplungseinrichtung, wobei der Instrumentenhalter einen Körper und einen Instrumententräger umfasst, der beweglich am Körper angebracht ist und eine Schnittstelle aufweist, die mit dem chirurgischen Instrument koppelbar ist, um das Instrument lösbar an dem Instrumentenhalter anzubringen, wobei die Antriebsanordnung mit dem Instrumentenhalter funktionsgekoppelt ist, um das Instrument mit mindestens zwei Freiheitsgraden zu versehen, welche die Rotation des chirurgischen Instruments gegenüber der Trägerbasis und die axiale Bewegung des chirurgischen Instruments gegenüber der Trägerbasis umfassen, und wobei die Antriebsanordnung einen ersten regelbaren bzw. steuerbaren Motor, der mit dem Instrumententräger funktionsverbunden ist, um ihn zu bewegen, und einen zweiten steuer- bzw. regelbaren Motor umfasst, der mit dem Instrumentenhalterkörper funktionsverbunden ist, um diesen gegenüber der Trägerbasis zu bewegen, wobei die Kopplungseinrichtung zum lösbaren Befestigen des Instrumentenhalters an der Trägerbasis und der Antriebsanordnung ausgebildet ist, und wobei der Instrumentenhalter zur Sterilisierung von der Trägerbasis und der Antriebsanordnung entfernbar ist.

Aus der US 2011/0282356 A1 ist ein ist ein Manipulator für Instrumente bekannt, mit einer Mehrzahl unabhängiger Antriebsmodule, von denen jedes einen Aktuatorausgang aufweist, wobei jeder Aktuatorausgang dazu ausgebildet ist, einen zugehörigen Aktuatoreingang eines chirurgischen Instruments unabhängig und ohne Krafteintrag von einem anderen Aktuatorausgang anzutreiben, wobei ein Gestell vorgesehen ist, das die Mehrzahl der unabhängigen Antriebsmodule beherbergt, wobei das Gestell ein distales Ende aufweist, von dem sich jeder der Aktuatorausgänge nach distal erstreckt, um an den zugehörigen Aktuatoreingang anzugreifen.

Aus der DE 10 2012 008 537 A1 ist eine Antriebseinheit für eine Instrumentenanordnung zur Befestigung an einem Roboter eines Chirurgierobotersystems bekannt, mit zumindest einem Drehantrieb mit einer Antriebswelle und mit einem Koppelteil zur Koppelung mit einer Antriebswelle des Instruments, wobei eine Instrumenten-Schnittstelle zwischen der Antriebseinheit und dem Instrument angeordnet ist, und wobei die Instrumenten-Schnittstelle eine Hülle zum Umhüllen der Antriebseinheit der Instrumentenanordnung aufweist, die mit einer schlauchartigen Innendurchführung versehen ist.

Aus der EP 2 889 009 A2 ist ein elektromechanisches Chirurgie-System bekannt, das eine handgeführte Chirurgie-Einheit mit einem Gehäuse und zumindest einer im Gehäuse gelagerten drehbaren Antriebswelle und eine Anschlussbaugruppe aufweist, die mit dem Gehäuse der Chirurgie-Einheit und einer Lasteinheit verbindbar ist, wobei die Lasteinheit zumindest ein axial verschiebbares Antriebselement aufweist, wobei die Anschlussbaugruppe ein Gehäuse, ein äußeres Rohr und zumindest eine Übertragungsbaugruppe zur Übertragung von Drehbewegungen und/oder Drehmomenten aufweist, und wobei die Übertragungsbaugruppe jeweils eine Antriebswelle und ein axial verschiebbares Antriebselement verbindet.

Seit geraumer Zeit befinden sich Systeme für robotische Operationen, insbesondere auf dem Gebiet der Telemedizin, im Stadium der Forschung und Erprobung. In der Vergangenheit wurde üblicherweise der Ansatz verfolgt, bekannte Funktionsprinzipien aus dem Gebiet der (industriellen) Robotik bzw. der industriellen Fertigung, Montage und Handhabung auf die Medizintechnik zu übertragen. Derartige Ansätze stoßen jedoch dann an Grenzen, wenn es um die besonderen Anforderungen auf dem Gebiet der minimal-invasiven Chirurgie geht.

Chirurgische Instrumente für minimal-invasive Eingriffe weisen üblicherweise eine langgestreckte Gestaltung bei sehr kleinem Durchmesser auf. Gleichwohl besteht ein Bedarf an Instrumenten, die im Inneren des Körpers erweiterte Funktionen ausüben können. Hierzu gehört etwa eine Verschwenkbarkeit um zumindest eine Schwenkachse sowie die Betätigung chirurgischer Werkzeuge, die am distalen Ende der Instrumente angebracht sind. Ein üblicher Durchmesser von Instrumenten für minimal-invasive Eingriffe beträgt etwa 10 mm (Millimeter). Diese Bauraumrestriktion stellt hohe Anforderungen an mechanische, optische sowie elektronische Komponenten für solche Instrumente.

Oftmals werden bei einem minimal-invasiven Eingriff eine Mehrzahl von Instrumenten verwendet. Dies kann auch gleichzeitig erfolgen. Mit anderen Worten werden häufig zwei oder gar mehr Instrumente gleichzeitig in das Innere des Patienten eingeführt. Beispielhaft kann dies etwa die gleichzeitige Verwendung eines endoskopischen Instruments zur Beobachtung sowie eines chirurgischen Instruments für den eigentlichen Eingriff umfassen.

Ferner sind sog. Single-Port-Operationen sowie Multi-Port-Operationen bekannt. Bei Single-Port-Operationen erfolgte der Eingriff über lediglich eine Öffnung im Körper. Bei Multi-Port-Operationen werden mehrere Körperöffnungen verwendet. Allgemein wird angestrebt, nur möglichst wenige Körperöffnungen zu verwenden, um die Belastung des Patienten zu minimieren. Wenn jedoch zwei oder mehr Instrumente gleichzeitig in das Körperinnere des Patienten eingeführt werden, und dies lediglich über einen einzigen Zugang erfolgt, erhöhen sich die Bauraumrestriktionen noch weiter. Bekannte Manipulatoren oder Roboter für medizinische Anwendungen stoßen dann oft an Grenzen. Dies ist zuweilen dadurch bedingt, dass die gleichzeitig einzuführenden Instrumente (außerhalb des Körpers) schlichtweg nicht nahe genug aneinander geführt bzw. zueinander ausgerichtet werden können, um bspw. parallel in das Körperinnere einzudringen.

Beispielsweise sind aus der DE 10 2012 211 886 A1 medizinische Instrumente für operative Eingriffe bekannt, die um mehr als eine Achse verschwenkbar sind. Auf diese Weise lässt sich die Bewegungsfreiheit im Körperinneren deutlich erhöhen. Allgemein besteht ein Bedarf an Instrumenten mit einer erhöhten Anzahl an Freiheitsgraden. Freiheitsgrade können bspw. Bewegungsfreiheitsgrade betreffen (Längsbewegungen, Schwenkbewegungen, Rotationen). Freiheitsgrade können jedoch auch die Betätigung chirurgischer Werkzeuge (Scheren, Klemmen, etc.) betreffen, die wiederum auf Bewegungen zurückzuführen sind. Je mehr Freiheitsgrade bei einem Instrument verwirklicht werden, desto mehr Pfade zur Bewegungsübertragung bzw. zur Übertragung von Kräften und/oder Momenten müssen berücksichtigt werden. In einem Instrumentenschaft oder Instrumentenarm können Bewegungen üblicherweise mittels Zug/Druck oder durch Drehbewegungen übertragen werden. Je komplexer das Instrument gestaltet ist, und je mehr Freiheitsgrade vorhanden sind, desto aufwändiger und schwergängiger ist die Bedienung des Instruments. Motorisch unterstützte oder motorisch gestützte Manipulationssysteme für chirurgische Eingriffe können somit auch dann von Vorteil sein, wenn es nicht oder nicht ausschließlich um den Aspekt der Telemedizin geht.

Medizinische Manipulationssysteme für Instrumente mit mehreren Freiheitsgraden (etwa zwei, drei oder vier Freiheitsgrade im Instrument selbst) können die Bedienung des Instruments deutlich vereinfachen, da eine rein mechanische Betätigung zum einen sehr kraftaufwändig ist und zum anderen koordinativ sehr herausfordernd ist. Demgemäß kann dem Chirurgen oder Operateur ein Arbeitsplatz zur Verfügung gestellt werden, der entsprechende Eingabeelemente aufweist, etwa Joysticks, Dummy-Instrumente bzw. Phantom-Instrumente oder Ähnliches. Auf diese Weise kann der Operateur Bewegungssignale erzeugen, die von einer Steuervorrichtung des Manipulationssystems in Steuersignale für Antriebe überführt werden, die diese Signale wiederum in Bewegungen des Instruments überführen.

Der Erfindung liegt die Aufgabe zugrunde, eine Handhabungsvorrichtung für chirurgische Instrumente sowie ein Manipulationssystem mit einer derartigen Handhabungsvorrichtung anzugeben, die für chirurgische Instrumente oder medizinische Instrumente mit erweitertem Funktionsumfang geeignet sind, insbesondere für Instrumente mit einer erhöhten Anzahl an (mechanischen) Freiheitsgraden bzw. Bewegungsfreiheitsgraden. Möglichst soll dabei die Eignung für Single-Port-Operationen verbessert werden. Vorzugsweise ist die Handhabungsvorrichtung sowie ein damit versehenes Manipulationssystem für die gleichzeitige Verwendung von zumindest zwei Instrumenten geeignet, die gleichzeitig in den Körper eines zu behandelnden Patienten eingeführt sind. Ferner ist es bevorzugt, wenn die Handhabungsvorrichtung selbst den Funktionsumfang des Instruments weiter erhöht, etwa durch Bereitstellung zumindest eines weiteren (Bewegungs-)Freiheitsgrades. Darüber hinaus soll die Handhabungsvorrichtung in einfacher Weise das Wechseln von Instrumenten ermöglichen. Im Betrieb selbst soll die Handhabungsvorrichtung ein reproduzierbares und vorhersagbares Verhalten der Instrumente während der Operation ermöglichen. Dies betrifft insbesondere eine hohe absolute Positioniergenauigkeit und eine hohe relative Positioniergenauigkeit bzw. Wiederholgenauigkeit.

Die Handhabungsvorrichtung betreffend wird die Aufgabe der Erfindung durch eine Handhabungsvorrichtung zur Aufnahme und Handhabung von Instrumenten für minimal-invasive Eingriffe gelöst, die ein Gestell und zumindest einen Instrumententräger mit einem Aufnahmeabschnitt zur Aufnahme eines Instrumentenarms aufweist, wobei der Instrumententräger beweglich am Gestell aufgenommen ist und mit zumindest einem Antrieb zu dessen Bewegung koppelbar ist, wobei der Instrumententräger zumindest abschnittsweise um seine Längsachse relativ zum Gestell rotierbar ist, wobei der Aufnahmeabschnitt eine Antriebsschnittstelle zur Übertragung mechanischer Energie zum Instrumentenarm aufweist, wobei die Antriebsschnittstelle zumindest einen Übertragungsanschluss zur Bewegungsübertragung, insbesondere zur Momentenübertragung, umfasst, wobei dem Übertragungsanschluss ein Instrumentenantrieb zugeordnet ist, wobei der Instrumententräger zumindest abschnittsweise entlang seiner Längserstreckung einen Übertragungsabschnitt aufweist, in dem zumindest ein Übertragungselement angeordnet ist, das eine Bewegungsübertragung zwischen dem Instrumentenantrieb und dem Übertragungsanschluss erlaubt, wobei das zumindest eine Übertragungselement konzentrisch zur Längsachse des Instrumententrägers ausgerichtet ist, und wobei der Übertragungsanschluss exzentrisch zur Längsachse angeordnet ist, so dass der Übertragungsanschluss während der Rotation des Instrumententrägers um dessen Längsachse verschwenkt wird.

Die Aufgabe der Erfindung wird auf diese Weise vollkommen gelöst. Die Erfindung wird durch den Schutzumfang des unabhängigen Anspruchs 1 definiert. Weitere bevorzugte Ausführungsformen sind in den abhängigen Ansprüchen offenbart.

Erfindungsgemäß gestattet nämlich die Handhabungsvorrichtung in einfacher Weise die Übertragung eines Antriebsimpulses (insbesondere eines Antriebsmoments) zu einem Instrument, das am Instrumententräger aufgenommen ist, obwohl der Instrumententräger gemeinsam mit einem daran aufgenommenen Instrument relativ zum Gestell um seine Längsachse (bzw. die Längsachse des Instrumentenarms) verdrehbar ist. Auf diese Weise kann die Handhabungsvorrichtung einerseits einen weiteren Bewegungsfreiheitsgrad, nämlich eine (globale) Rotation des Instruments um die Längsachse bereitstellen. Gleichwohl können innerhalb des Instrumentenarms die gewünschten Bewegungen übertragen werden, ohne dass sich die (globale) Verdrehbarkeit des Instruments nachteilig auswirkt. Der Instrumentenarm kann üblicherweise auch als Instrumentenschaft bezeichnet werden. Der Instrumentenarm weist üblicherweise eine Längserstreckung auf, die ein Mehrfaches oder gar Vielfaches des Durchmessers beträgt.

Die Handhabungsvorrichtung gemäß dem obigen Aspekt eignet sich insbesondere für Instrumente, bei denen der Antrieb für zumindest einen Bewegungsfreiheitsgrad, vorzugsweise für zwei oder mehr Bewegungsfreiheitsgrade, im Instrument durch Momentenübertragung erfolgt. Mit anderen Worten werden für diese Freiheitsgrade im Instrument keine Schubstangen zur Übertragung von der Handhabungsvorrichtung zum Instrument benötigt. Die Übertragung eines Drehmoments, etwa über Torsionsstäbe, flexible oder starre Wellen, oder dergleichen, ist insbesondere dann von Vorteil, wenn das Instrument zumindest einen, vorzugsweise mehrere, verschwenkbare Abschnitt(e) aufweist, so dass es sich bei zumindest einem, vorzugsweise zwei oder mehreren der Bewegungsfreiheitsgrade um Rotationsfreiheitsgrade (bzw. Schwenkfreiheitsgrade) handelt. Eine reine Betätigung über Schubstangen würde bei einer Bewegung um eine Achse unter Umständen zu einer Rückkopplung führen, die eine Bewegung einer anderen Achse induziert, die nicht gewünscht ist. Derartige Nachteile können konstruktiv verringert oder gar beseitigt werden. Dies ist jedoch mit einem sehr hohen Aufwand verbunden.

Der zumindest eine Übertragungsanschluss, der das Drehmoment an das Instrument überträgt, ist exzentrisch zur Längsachse des Instrumententrägers angeordnet. Demgemäß wird der Übertragungsanschluss während der Verdrehung des Instrumententrägers um dessen Längsachse insgesamt verschwenkt, dies muss keine Rotation des Übertragungsanschlusses um seine (eigene) Längsachse umfassen. Vorzugsweise ist das zumindest eine Übertragungselement derart mit dem Übertragungsanschluss gekoppelt, dass der Übertragungsanschluss während einer Verdrehung des Instrumententrägers um die Längsachse betragsgleich und richtungsgleich mitbewegt wird

Vorzugsweise erfolgt die Bewegungsübertragung am bzw. im Instrumententräger spielarm bzw. nahezu spielfrei. Dies kann insbesondere eine Bewegungsübertragung über hinreichend starre bzw. steife Elemente umfassen, also nicht über nachgiebige Elemente (wie etwa Ausgleichskupplungen, flexible Wellen, Drahtseile oder Ähnliches). Stattdessen ist es bevorzugt, wenn zur Bewegungsübertragung bei der Handhabungsvorrichtung Zahnradstufen verwendet werden, die über steife Wellen oder Rohre miteinander gekoppelt sind. Dies kann insgesamt zur Erhöhung der Genauigkeit der Betätigung des Instruments beitragen.

Allgemein ist der Instrumententräger dazu ausgestaltet, das Instrument aufzunehmen, sowie Antriebsmomente zur Betätigung des Instruments am distalen Ende des Instrumententrägers in das proximale Ende des Instrumentenschaftes zu übertragen. Dies beinhaltet, dass erforderliche Antriebe (Instrumentenantriebe) für die Freiheitsgrade des Instruments beabstandet vom proximalen Ende des Instrumentenschafts oder -arms an der Handhabungsvorrichtung angeordnet sein können, da der Instrumententräger einen Abstand zwischen den Antrieben und dem Instrument überbrückt.

Vorzugsweise weist das Instrument zwei, drei oder vier (interne) Bewegungsfreiheitsgrade auf, die etwa Schwenkbewegungen sowie Betätigungen von Werkzeugen und Ähnliches umfassen können. Vorzugsweise stellt die Handhabungsvorrichtung mittelbar über den Instrumententräger zumindest zwei weitere (globale) Bewegungsfreiheitsgrade für das Instrument bereit, insbesondere einen Rotationsfreiheitsgrad um die Längsachse (des Instrumententrägers) sowie eine Schubbewegung entlang der Längsachse bzw. parallel zur Längsachse. Bewegungen, die von diesen Freiheitsgraden Gebrauch machen, haben vorzugsweise keine Auswirkungen auf (lokale) Zustände bzw. Bewegungen des Instruments selbst. Mit anderen Worten ist vorzugsweise eine lokale Bewegungsreferenz des Instruments von einer globalen Bewegungsreferenz unabhängig, und zwar auch dann, wenn die Antriebe für das Instrument zumindest mittelbar gestellfest aufgenommen sind.

In bevorzugter Weiterbildung der Handhabungsvorrichtung weist die Antriebsschnittstelle des zumindest einen Instrumententrägers zumindest zwei, vorzugweise zumindest drei oder vier, Übertragungsanschlüsse auf, von denen zumindest zwei von der Längsachse radialversetzt und voneinander beabstandet angeordnet sind, wobei im Übertragungsabschnitt zumindest zwei, vorzugweise zumindest drei oder vier, Übertragungselemente angeordnet sind, die den zumindest zwei Übertragungsanschlüssen zugordnet sind, wobei zumindest zwei, vorzugweise zumindest drei oder vier, Instrumentenantriebe vorgesehen sind, wobei die Handhabungsvorrichtung einen ersten Bewegungsfreiheitsgrad für ein aufgenommenes Instrument bereitstellt, dem ein erster Instrumentenantrieb, ein erstes Übertragungselement und ein erster Übertragungsanschluss zugeordnet sind, die zur Drehübertragung miteinander gekoppelt sind, insbesondere durch Zahnradstufen, wobei die Handhabungsvorrichtung einen zweiten Bewegungsfreiheitsgrad für ein aufgenommenes Instrument bereitstellt, dem ein zweiter Instrumentenantrieb, ein zweites Übertragungselement und ein zweiter Übertragungsanschluss zugeordnet sind, die zur Drehübertragung miteinander gekoppelt sind, insbesondere durch Zahnradstufen, und wobei das erste Übertragungselement und das zweite Übertragungselement konzentrisch zur Längsachse des Instrumententrägers ausgerichtet und unabhängig voneinander antreibbar sind, wobei das zweite Übertragungselement rohrartig gestaltet ist und das erste Übertragungselement zumindest abschnittsweise umschließt.

Mit anderen Worten ist im Übertragungsabschnitt eine Mehrzahl von Übertragungselementen für die instrumentenseitigen Bewegungsfreiheitsgrade angeordnet, von denen zumindest zwei Übertragungselemente, vorzugsweise sämtliche Übertragungselemente, konzentrisch zueinander ausgerichtet sind. Auf diese Weise verändert sich bei einer (globalen) Verdrehung des Instrumentenarms oder -schaftes bei einer Verdrehung des Instrumententrägers die Relativlage zwischen den Übertragungselementen nicht. Deshalb können die Instrumentenantriebe, insbesondere deren Motoren, die mittelbar oder unmittelbar am Gestell festgelegt sind, bei der (globalen) Drehung des Instruments an ihrer Position verbleiben und müssen nicht mit um die Längsachse des Instrumentenschafts gedreht oder rotiert werden. Dies führt zu wesentlichen Bauraumeinsparungen und einer deutlichen Verringerung des Herstellungsaufwands.

Mit anderen Worten ähnelt die Funktion des Instrumententrägers der einer hydraulischen Drehdurchführung, wobei der Instrumententräger gewissermaßen eine "mechanische Drehdurchführung" bereitstellt. Die zumindest abschnittsweise konzentrische Gestaltung des Instrumententrägers erlaubt eine Kraftübertragung bzw. Momentenübertragung entlang mehrerer Pfade, und zwar ohne unerwünschte Wechselwirkungen, Interferenzen oder Ähnlichem zwischen den Pfaden.

Gemäß einer vorteilhaften Weiterbildung stellt die Handhabungsvorrichtung ferner einen dritten Bewegungsfreiheitsgrad und einen vierten Bewegungsfreiheitsgrad für das aufgenommene Instrument bereit, wobei dem dritten Bewegungsfreiheitsgrad ein dritter Instrumentenantrieb, ein drittes Übertragungselement und ein dritter Übertragungsanschluss zugeordnet sind, die zur Drehübertragung miteinander gekoppelt sind, insbesondere durch Zahnradstufen, wobei dem vierten Bewegungsfreiheitsgrad ein vierter Instrumentenantrieb, ein viertes Übertragungselement und ein vierter Übertragungsanschluss zugeordnet sind, die zur Drehübertragung miteinander gekoppelt sind, insbesondere durch Zahnradstufen, und wobei das dritte Übertragungselement und das vierte Übertragungselement konzentrisch zur Längsachse des Instrumententrägers ausgerichtet und unabhängig voneinander antreibbar sind, wobei das vierte Übertragungselement rohrartig gestaltet ist und das dritte Übertragungselement zumindest abschnittsweise umschließt, wobei das dritte Übertragungselement rohrartig gestaltet ist und das zweite Übertragungselement zumindest abschnittsweise umschließt.

Insgesamt kann die Handhabungsvorrichtung somit fünf oder sechs Bewegungsfreiheitsgrade für das Instrument bereitstellen. Dies kann vier (interne) Bewegungsfreiheitsgrade für das Instrument umfassen, die eine Betätigung des Instruments über die vier Übertragungsanschlüsse umfassen. Daneben sind zumindest zwei weitere (globale) Bewegungsfreiheitsgrade denkbar, etwa eine Verdrehung des Instruments um die Längsachse sowie eine translatorische Bewegung entlang der Längsachse.

Gemäß einer weiteren Ausgestaltung sind die Übertragungsanschlüsse um die Längsachse verteilt und exzentrisch zur Längsachse angeordnet. Dies kann eine revolverartige Anordnung der Übertragungsanschlüsse umfassen. Gemäß einer beispielhaften Ausführung definieren die Übertragungsanschlüsse (mit ihren jeweiligen Achsen) insgesamt einen Umfangskreis um die Längsachse. Gleichermaßen weist dann also das Instrument eine entsprechende Anzahl an Instrumenteneingängen auf, die mit den Übertragungsanschlüssen koppelbar sind, wenn das Instrument am Instrumententräger aufgenommen ist. Es versteht sich, dass etwa dann, wenn beim Instrument selbst weniger Bewegungsfreiheitsgrade vorhanden sind, als die Handhabungsvorrichtung bereitstellt, am Instrument eine geringere Anzahl von Instrumenteneingängen vorgesehen ist. Dies ist nicht von Nachteil, da der entsprechende Bewegungsfreiheitsgrad auf Seiten der Handhabungsvorrichtung von den übrigen Bewegungsfreiheitsgraden entkoppelt ist.

Gemäß einer weiteren Ausgestaltung der Handhabungsvorrichtung ist das zumindest eine Übertragungselement, vorzugsweise jedes der Übertragungselemente im Übertragungsabschnitt, schaftartig oder rohrartig gestaltet und mit einer eingangsseitigen, proximalen Zahnradstufe und einer ausgangsseitigen, distalen Zahnradstufe, gekoppelt.

Insgesamt kann sich also eine konzentrische Anordnung aus mehreren ineinander aufgenommenen Rohren ergeben. Es versteht sich, dass die Zahnradstufen zum Antrieb und Abtrieb der Übertragungselemente von den entsprechenden Zahnradstufen der anderen Übertragungselemente axial versetzt sind. Die Zahnräder der Übertragungselemente sind konzentrisch zur Längsachse des Instrumententrägers angeordnet.

Gemäß einer beispielhaften Ausgestaltung ist das erste Übertragungselement innen angeordnet und vorzugsweise als Vollwelle ausgeführt. Das zweite Übertragungselement ist rohrartig gestaltet und umschließt das erste Übertragungselement. Das dritte Übertragungselement ist rohrartig gestaltet und umschließt das zweite Übertragungselement. Das vierte Übertragungselement ist rohrartig gestaltet und umschließt das dritte Übertragungselement. Es versteht sich, dass auch weitere Übertragungselemente denkbar sind, die rohrartig gestaltet sind und die übrigen Übertragungselemente konzentrisch umschließen. Bei der Gestaltung mit vier Übertragungselementen weist das erste (innere) Übertragungselement zwangsläufig die größte axiale Erstreckung zwischen dem proximalen Zahnrad und dem distalen Zahnrad auf. Die axiale Erstreckung zwischen ihren proximalen und distalen Zahnrädern betreffend, folgen das zweite Übertragungselement, das dritte Übertragungselement und das vierte Übertragungselement, wobei das vierte (äußere) Übertragungselement die kürzeste axiale Erstreckung aufweist.

Gemäß einer weiteren Ausgestaltung ist das zumindest eine Übertragungselement zur Momentenübertragung an seinem proximalen Ende mit dem Instrumentenantrieb und an seinem distalen Ende mit dem Übertragungsanschluss gekoppelt, wobei bei einer Verdrehung des Instrumententrägers um die Längsachse, die eine betragsgleiche und richtungsgleiche Schwenkbewegung des Übertragungsanschlusses bewirkt, der jeweils zugeordnete Instrumentenantrieb in seiner definierten Relativlage bezüglich des Gestells verbleibt. Dies trifft vorzugsweise auf die Instrumentenantriebe des ersten, zweiten, dritten und vierten Bewegungsfreiheitsgrades zu.

Gemäß einer weiteren Ausgestaltung der Handhabungsvorrichtung ist am Gestell zumindest eine Längsführung ausgebildet, an der ein Schlitten aufgenommen ist, der den zumindest einen Instrumententräger trägt, wobei der zumindest eine Instrumententräger gemeinsam mit dem Schlitten relativ zum Gestell verfahrbar ist. Auf diese Weise ist ein weiterer (fünfter) Bewegungsfreiheitsgrad verwirklicht, insbesondere ein translatorischer Bewegungsfreiheitsgrad. Zumindest gemäß einigen Ausgestaltungen ist die Längsführung als Vertikalführung gestaltet. Es versteht sich, dass jedoch auch andere Orientierungen der Längsführung vorstellbar sind. Eine translatorische Bewegung des Instrumententrägers entlang der Längsführung bewirkt eine betragsgleiche und richtungsgleiche Bewegung des aufgenommenen Instruments.

Gemäß einer weiteren Ausgestaltung der Handhabungsvorrichtung ist der zumindest eine Instrumentenantrieb, ist vorzugsweise jeder Instrumentenantrieb, der dem Instrumententräger zugeordnet ist, am Schlitten aufgenommen. Mit anderen Worten werden demgemäß auch die Instrumentenantriebe bei der Längsbewegung des Schlittens gemeinsam mit dem Instrumententräger verfahren. Gemäß einer weiteren Ausgestaltung sind die Instrumentenantriebe, sofern eine Mehrzahl von Instrumentenantrieben am Schlitten aufgenommen ist, umfänglich des Instrumententrägers und parallel zu diesem orientiert angeordnet. Insbesondere können die Instrumentenantriebe zumindest abschnittsweise umfänglich des Übertragungsabschnitts angeordnet sein. Dies kann den erforderlichen Bauraum weiter verringern.

Beispielhaft sind auch die Instrumentenantriebe schaftartig oder zylindrisch gestaltet und weisen eine Längserstreckung auf, die größer als der Durchmesser ist. An die Motoren können geeignete Getriebe angeflanscht sein. Beispielhaft eignen sich positionsgesteuerte bzw. positionsgeregelte Motoren. Hierbei kann es sich um sog. Servomotoren handeln, etwa um bürstenlose DC-Servomotoren (Gleichstrom-Servomotoren). Andere positionsgesteuerte Motoren sind denkbar. Da für jeden der instrumentenseitigen Bewegungsfreiheitsgrade ein separater Instrumentenantrieb vorgesehen ist, können die Antriebe gemäß einem Ausführungsbespiel parallel zur Längsachse ausgerichtet und um den Instrumententräger zu angeordnet bzw. gruppiert werden. Es ist vorteilhaft, die Instrumentenantriebe (insbesondere deren Motoren) direkt auf dem Schlitten aufzunehmen, da auf diese Weise kurze Übertragungswege ermöglicht sind, die sich in einer erhöhten Genauigkeit und in verringertem Spiel niederschlagen.

Gemäß einer beispielhaften Weiterbildung dieser Ausgestaltung sind die Instrumentenantriebe um den Instrumententräger versetzt zu diesem am Schlitten angeordnet, wobei der Instrumententräger in einem Randbereich des Schlittens angeordnet ist. Vorzugsweise ist der Instrumententräger an einem seitlichen Rand des Schlittens angeordnet. Dies kann dazu beitragen, dass der Instrumententräger und das an diesem aufgenommenen Instrument in dieser Richtung (seitlich) sehr nahe an eine gewünschte Zielposition herangefahren werden kann, etwa sehr nahe an einen weiteren Instrumententräger, an dem ein weiteres Instrument aufgenommen ist. Dies kann die Eignung der Handhabungsvorrichtung für Single-Port-Operationen weiter erhöhen.

Gemäß einer weiteren Weiterbildung sind zumindest zwei Instrumentenantriebe auf gegenüberliegenden Seiten des Schlittens aufgenommen. Auch gemäß dieser Ausgestaltung sind die Instrumentenantriebe parallel zum Instrumententräger ausgerichtet. Da jedoch der Instrumententräger selbst aufgrund der konzentrischen Anordnung der Übertragungselemente eine gewisse (bauartbedingte) Längserstreckung aufweist, kann es von Vorteil sein, die Instrumentenantriebe derart anzuordnen und auszurichten, dass die Längserstreckung maximal genutzt wird. Dies kann dazu beitragen, den Bauraum in Querrichtungen zur Längserstreckung (insbesondere seitliche Richtungen) zu minimieren. Insgesamt können die Instrumentenantriebe, bei Nutzung von vier Instrumentenfreiheitsgraden, eine H-förmige Anordnung bezüglich einer Basis des Schlittens einnehmen.

Gemäß einer weiteren Ausgestaltung der Handhabungsvorrichtung ist der Schlitten mit einem Längsantrieb gekoppelt. Vorzugsweise ist der Längsantrieb als Gewindetrieb oder Kugelgewindetrieb ausgestaltet. Diese Maßnahme kann dadurch weitergebildet sein, dass der Längsantrieb einen gestellfest aufgenommenen Motor umfasst, der eine Spindel antreibt. Demgemäß ist beispielhaft am Schlitten eine Spindelmutter aufgenommen, die bei einer Rotation der Spindel in der Längsrichtung verfahren wird und den Schlitten mitnimmt. Der Instrumententräger und ein daran aufgenommenes Instrument werden mit dem Schlitten durch den Längsantrieb in der Längsrichtung bewegt.

Gemäß einer weiteren Ausgestaltung der Handhabungsvorrichtung ist am Schlitten ferner ein Rotationsantrieb aufgenommen, der eine Rotation des Instrumententrägers um seine Längsachse bewirkt, wobei die Rotation des Instrumententrägers eine Drehmitnahme des zumindest einen Übertragungsanschlusses um die Längsachse bewirkt. Mit dem Längsantrieb wird ein fünfter Bewegungsfreiheitsgrad für das Instrument bereitgestellt. Mit dem Rotationsantrieb wird ein sechster Bewegungsfreiheitsgrad für das Instrument bereitgestellt. Es versteht sich, dass die Handhabungsvorrichtung selbst, in zumindest einigen Ausgestaltungen, wiederum an einem Manipulator, Roboter oder einer anders gestalteten (globalen) Bewegungseinrichtung aufgenommen sein kann.

Gemäß einer weiteren Ausgestaltung der Handhabungsvorrichtung weist der Aufnahmeabschnitt des zumindest einen Instrumententrägers eine Kontaktschnittstelle zur Übertragung elektrischer Energie oder elektrischer Signale auf, wobei die Kontaktschnittstelle vorzugsweise in einem Zentrum des Aufnahmeabschnitts angeordnet ist. Zu diesem Zweck kann etwa ein geeigneter Stecker oder eine geeignete Buchse vorgesehen sein. Vorzugsweise weist die Kontaktschnittstelle eine Mehrzahl von Kontakten auf. Instrumentenseitig können bspw. Sensoren vorgesehen sein, deren Signale über die Kontaktschnittstelle übermittelt werden. Es ist jedoch auch vorstellbar, dass instrumentenseitig Aktuatoren, Bildgeber, Einrichtungen zur Erzeugung thermischer Energie, oder Ähnliches ausgebildet sind. Demgemäß können vielfältige Signale über die Kontaktschnittstelle übertragen werden. Die Kontaktschnittstelle bewegt sich bei einer Verdrehung des Instrumententrägers mit dem Instrumententräger und mit dem daran aufgenommenen Instrument. Demgemäß kann eine Kontaktierung der Gestellseite der Handhabungsvorrichtung (bzw. des übergeordneten Manipulationssystems) über flexible Anschlüsse erfolgen, etwa über einen Kabelschlepp oder dergleichen. Dies ist insbesondere dann denkbar, wenn der Instrumententräger selbst nur um ein bestimmtes Maß um seine Längsachse verdrehbar ist. Alternativ ist es jedoch vorstellbar, Schleifkontakte, Ringkontakte oder Ähnliches zwischen dem Instrumententräger und dem Gestell vorzusehen, um unabhängig von einer absoluten Drehlage des Instrumententrägers elektrische Signale bzw. elektrische Energie übertragen zu können.

Gemäß einer weiteren Ausgestaltung der Handhabungsvorrichtung ist der Aufnahmeabschnitt des zumindest einen Instrumententrägers als Rastaufnahme für ein proximales Ende eines Instruments, insbesondere eines Instruments für die minimal-invasive Chirurgie oder Diagnose, ausgebildet, wobei am Aufnahmeabschnitt zumindest ein Drehlagesicherungselement ausgebildet ist, das im eingerückten Zustand mit einem Gegenelement eines Instrumentenarms zusammenwirkt, um eine definierte Drehlage zwischen dem Instrumentenarm und dem Instrumententräger zu bewirken. Die Drehlagesicherung kann über geeignete Vorsprünge, Vertiefungen, Verzahnungen oder Ähnliches bewirkt werden. Die Drehlagesicherung trägt zur Verhinderung einer Fehlmontage bei. Vorzugsweise ist das Instrument drehfest am Aufnahmeabschnitt des Instrumententrägers aufgenommen, um gemeinsam mit diesem um die Längsachse verdreht zu werden.

Gemäß einer Weiterbildung dieser Ausgestaltung ist am Aufnahmeabschnitt eine Mehrzahl von Rastelementen, insbesondere von Rastkugeln, aufgenommen, die im eingerasteten Zustand in zumindest eine Rastausnehmung am Instrumentenarm eingreifen. Die Rastausnehmung ist beispielhaft als umlaufende Rastnut ausgestaltet. Auch Gestaltungen mit einer Mehrzahl von Rastausnehmungen, die jeweils kugelabschnittsförmige Vertiefungen ausbilden, sind denkbar. Die Rastaufnahme kann ferner eine axial verschiebbare Rasthülse umfassen, die am Aufnahmeabschnitt aufgenommen ist und gegen eine Vorspannkraft verschiebbar ist, um ein Ausrücken der Rastelemente aus dem eingerasteten Zustand zu erlauben. Auf diese Weise kann der Instrumentenarm einfach und sicher am Instrumententräger aufgenommen bzw. von diesem entfernt werden. Ein unbeabsichtigtes Lösen des Instrumentenarms wird durch eine Vorspannung der Rasthülse unterbunden. Die Rastelemente sind vorzugsweise radial verschiebbar, um selektiv eine formschlüssige Lagesicherung zwischen dem Aufnahmeabschnitt und dem Instrumentenarm zu bewirken. Vorzugsweise weist die Rasthülse einen (flachen) Kegelwinkel auf, der im eingerasteten Zustand des Instrumentenarms eine Selbsthemmung der Rasthülse und der Rastelemente bewirkt.

Gemäß einer weiteren Ausgestaltung umfasst die Handhabungsvorrichtung zumindest einen ersten Instrumententräger und einen zweiten Instrumententräger, die beweglich am Gestell aufgenommen und zumindest abschnittsweise relativ zueinander beweglich sind, wobei jeder Instrumententräger mit einem Aufnahmeabschnitt zur Aufnahme eines Instrumentenarms versehen ist. Vorzugsweise sind die Instrumententräger parallel zueinander und einander benachbart am Gestell aufgenommen. Ein wesentlicher Vorteil der Handhabungsvorrichtung besteht darin, dass bei einer Verdrehung des Instrumententrägers um die Längsachse die Instrumentenantriebe selbst nicht mit verdreht bzw. mit verschwenkt müssen. Dies minimiert den erforderlichen Bauraum bzw. den erforderlichen Bewegungsraum. Somit besteht die Möglichkeit, mehrere Instrumententräger in unmittelbarer Nachbarschaft anzuordnen, wobei die Instrumentenantriebe den erreichbaren Mindestabstand zwischen den Instrumententrägern nicht oder nur unwesentlich erhöhen. Die Eignung der Handhabungsvorrichtung für Single-Port-Operationen kann sich weiter erhöhen. Gemäß einer Weiterbildung dieser Ausgestaltung ist der erste Instrumententräger an einem ersten Schlitten und der zweite Instrumententräger an einem zweiten Schlitten aufgenommen, wobei der erste Instrumententräger und der zweite Instrumententräger in einander zugewandten Randbereichen der Schlitten angeordnet sind, insbesondere einander unmittelbar benachbart.

Gemäß einer beispielhaften Weiterbildung sind Längsachsen des ersten Instrumententrägers und des zweiten Instrumententrägers um ein Versatzmaß a voneinander versetzt, das ein Verhältnis zu einem Bauraumdurchmesser D der Instrumententräger aufweist, das weniger als 3.5:1, vorzugsweise weniger als 2.5:1, weiter bevorzugt weniger als 1.5:1 beträgt. Es versteht sich, dass neben einer parallelen Anordnung des ersten Instrumententrägers und des zweiten Instrumententrägers auch eine zumindest teilweise zueinander geneigte Orientierung vorstellbar ist, um im Bereich der distalen Enden der Instrumente noch geringere effektive Abstände bewirken zu können.

Gemäß einer weiteren Ausgestaltung der Handhabungsvorrichtung ist zumindest ein Instrumentenantrieb mit einem positionsgeregelten Motor vorgesehen, der derart angesteuert ist, dass während der Verdrehung des Instrumententrägers um die Längsachse der mit dem Instrumentenantrieb gekoppelte Übertragungsanschluss eine lokale Ausgleichsbewegung um seine Achse ausführt, so dass eine lokale Drehwinkellage des Übertragungsanschlusses in Bezug auf den Instrumententräger beibehalten wird. Bei der Verdrehung des Instrumententrägers verbleiben die Instrumentenantriebe in ihrer Ursprungsposition. Auf diese Weise bewirkt die Verdrehung des Instrumententrägers mittelbar eine Verdrehung der Übertragungsanschlüsse, wenn eine Bewegungskopplung besteht. Jedoch können die Motoren der Instrumentenantriebe geeignet angesteuert werden, um eine Ausgleichsbewegung zu vollziehen. Aus Sicht des aufgenommenen Instruments ändert sich somit abgesehen von der (globalen) Verdrehung nichts am aktuellen Bewegungszustand (internes Instrumenten-Bezugssystem). Mit anderen Worten hat die Verdrehung des Instrumententrägers z.B. keine Auswirkung auf den Zustand eines Greifers am Instrument bzw. auf einen (Instrumenten-internen) Verschwenkwinkel zwischen Komponenten des Instruments.

Gemäß einer alternativen Ausgestaltung der Handhabungsvorrichtung ist zumindest ein Instrumentenantrieb mit einem rastmomentarmen Motor oder mit einer Kupplung vorgesehen, wobei abtriebsseitig des Übertragungselements (in gewissem Maße) Selbsthemmung auftritt, so dass während der Verdrehung des Instrumententrägers um die Längsachse der mit dem Instrumentenantrieb gekoppelte oder koppelbare Übertragungsanschluss seine lokale Drehwinkellage in Bezug auf den Instrumententräger beibehält. Mit anderen Worten würde gemäß dieser Ausgestaltung durch die Bewegung des Instrumententrägers und aufgrund der Selbsthemmung umgekehrt eine Rückkopplung hin zum antriebsseitigen (proximalen) Ende des Instrumententrägers bzw. der Instrumentenantriebe erfolgen.

Gemäß einer weiteren, alternativen Ausgestaltung kann eine unerwünschte Rückkopplung oder parasitäre Bewegung bzw. Mitbewegung der Übertragungselemente bei der Verdrehung des Instrumententrägers durch geeignete Auslegung der Getriebestufen für die jeweiligen Bewegungsfreiheitsgrade des Instruments vermieden werden. Wenn also bei der Verdrehung des Instrumententrägers die eingangsseitig des Übertragungselements vorgesehene Zahnradstufe eine definierte Verdrehbewegung des Übertragungselements bewirkt und die abtriebsseitig des Übertragungselements vorgesehene(n) Übertragungsstufe(n) eine betragsmäßig gleiche aber gegengerichtete Verdrehbewegung bewirkt/bewirken, ändert sich nichts an der relativen Drehorientierung des Übertragungsanschlusses gegenüber dem Instrumententräger bzw. gegenüber dem aufgenommenen Instrument.

Gemäß einer weiteren Ausgestaltung der Handhabungsvorrichtung weist der zumindest eine Übertragungsanschluss ein Mitnahmeprofil auf, das formschlüssig mit einem Gegenprofil eines instrumentenseitigen Instrumenteneingangs koppelbar ist, wobei das Mitnahmeprofil eine Ausrichtkontur zur Montagevereinfachung umfasst. Vorzugsweise bewirkt die Ausrichtkontur eine radiale und eine rotatorische Vorausrichtung zwischen dem Übertragungsanschluss und einem diesem zugeordneten Instrumenteneingang. Die radiale Vorausrichtung erfolgt in Bezug auf eine radiale Soll-Position gegenüber der Längsachse des Instrumententrägers. Die rotatorische Vorausrichtung erfolgt in Bezug auf eine Rotationsposition des Instrumenteneingangs bzw. des Übertragungsanschlusses selbst. Das heißt mit anderen Worten, eine Ist-Drehposition des instrumentenseitigen Instrumenteneingangs wird vorzugsweise an eine Ist-Drehposition des diesem zugeordneten Übertragungsanschlusses angepasst.

Demgemäß kann das Mitnahmeprofil Einführschrägen und dergleichen aufweisen, ferner jedoch auch Rampen oder ähnliche Schrägflächen zur Definition der relativen Drehlage zwischen dem Mitnahmeprofil und dem Gegenprofil. Die Rampen können eine Teilung definieren, die größer als eine Teilung des Mitnahmeprofils des Gegenprofils ist. Beispielhaft können Mitnahmeprofil bzw. Gegenprofil als Vierkantprofil ausgestaltet sein, also eine Teilung von 90° aufweisen. Demgemäß ist es vorstellbar, dass die Ausrichtkontur etwa lediglich zwei Rampen zur rotatorischen Vorausrichtung aufweist, die demgemäß eine Teilung von 180° definieren. Mit anderen Worten kann der Instrumenteneingang des Instruments nur in zwei Drehlagen mit dem Übertragungsanschluss gekoppelt werden. Die Bewegungsübertragung kann hingegen über ein Vielfaches an Flächen erfolgen, je nach Gestaltung des Mitnahmeprofils und des Gegenprofils. Es ist jedoch auch vorstellbar, dass das Instrument in vier Drehlagen, analog zur Teilung des Mitnahmeprofils bzw. Gegenprofils aufnehmbar ist, wenn nur jede zweite von vier instrumentenseitigen Gegenflächen eine der Rampen kontaktiert.

Gemäß einer Weiterbildung dieser Ausgestaltung umfasst das Mitnahmeprofil eine Mitnahmeausnehmung am distalen Ende des Übertragungsanschlusses, wobei in einem Stirnbereich des Mitnahmeprofils geneigte Versatzflächen angeordnet sind, die die Mitnahmeausnehmung umgeben. Beispielhaft kann das Mitnahmeprofil als Vierkantprofil, Torx-Profil, Zahnwellenprofil, Inbus-Profil oder in ähnlicher Weise gestaltet sein, wobei das Gegenprofil auf Seiten des Instrumenteneingangs korrespondierend gestaltet ist.

Gemäß einer weiteren Ausgestaltung weist die Antriebsschnittstelle eine Mehrzahl von Übertragungsanschlüssen auf, die im Aufnahmeabschnitt angeordnet sind, wobei zumindest einige der Übertragungsanschlüsse axial voneinander versetzt sind, so dass instrumentenseitige Instrumenteneingänge, die den Übertragungsanschlüssen zugeordnet sind, beim Fügen des Instrumentenarms nacheinander mit den Übertragungsanschlüssen gekoppelt werden. Dies kann die Aufnahme des Instruments am Instrumententräger und insbesondere das Verrasten deutlich vereinfachen.

Gemäß einer weiteren Ausgestaltung, die alternativ oder zusätzlich vorstellbar ist, ist der Instrumentenantrieb, der dem zumindest einen Übertragungsanschluss zugeordnet ist, dazu ausgestaltet, beim Fügen des Instrumentenarms den Übertragungsanschluss hin- und herschwenkend anzutreiben, um das Einrücken des Mitnahmeprofils und des Gegenprofils zu unterstützen. Dieser Vorgang wird auch als "Jiggeln" (Wackeln) bezeichnet. Vorzugsweise wird eine Mehrzahl von Übertragungsanschlüssen durch die ihnen zugeordneten Instrumentenantriebe zumindest abschnittsweise gleichzeitig hin- und herschwenkend angetrieben, wobei der Antrieb bei verschiedenen Drehgeschwindigkeiten erfolgt. Insgesamt kann sich also bei den Übertragungsanschlüssen eine Drehoszillationsbewegung bzw. eine angenäherte Drehoszillationsbewegung ergeben, wobei Oszillationsgeschwindigkeiten der einzelnen Übertragungsanschlüsse vorzugsweise voneinander abweichen. Dies kann das "Finden" des Gegenelements und insgesamt das Fügen und Verrasten des Instrumentenarms am Instrumententräger verkürzen und vereinfachen. Auf diese Weise wird insbesondere dem Umstand Rechnung getragen, dass das Fügen üblicherweise "blind" erfolgt, da keine freie Sicht auf die Fügestelle besteht.

Die Aufgabe der Erfindung wird ferner durch ein Manipulationssystem für minimal-invasive chirurgische Operationen, insbesondere durch ein Telemanipulationssystem für minimal-invasive Single-Port-Operationen, gelöst, das mit einer Ansteuerungsplattform versehen ist, die eine Handhabungsvorrichtung nach einem der vorstehend beschriebenen Aspekte aufweist. In zumindest einigen Ausgestaltungen besteht die Ansteuerungsplattform im Wesentlichen aus der Handhabungsvorrichtung. Es sind jedoch auch Ausgestaltungen vorstellbar, bei denen zusätzlich weitere Module vorgesehen sind, etwa zusätzliche Handhabungsvorrichtungen, Beobachtungsvorrichtungen, Beleuchtungsvorrichtungen, Aufnahmen für Mikroskope, Endoskope, Exoskope, oder dergleichen.

Weitere Merkmale und Vorteile der Erfindung ergeben sich aus der nachfolgenden Beschreibung bevorzugter Ausführungsbeispiele unter Bezugnahme auf die Zeichnungen. Es zeigen:
- Fig. 1: eine schematische, stark vereinfachte perspektivische Ansicht einer Operationsumgebung mit einem Telemanipulationssystem;
- Fig. 2: eine Detailansicht der Darstellung gemäß Fig. 1;
- Fig. 3: eine perspektivische seitliche Teilansicht einer Handhabungsvorrichtung gemäß zumindest einigen Aspekten der vorliegenden Offenbarung;
- Fig. 4: eine weitere Ansicht der Handhabungsvorrichtung gemäß Fig. 3 in einer abweichenden Orientierung;
- Fig. 5: noch eine weitere Ansicht der Handhabungsvorrichtung gemäß Fig. 4 in einer weiteren perspektivischen (frontalen) Orientierung, wobei Fig. 5 eine von der Darstellung gemäß den Figuren 3 und 4 abweichende Position eines Schlittens veranschaulicht;
- Fig. 6: eine schematische Seitenansicht einer gegenüber der Darstellung gemäß den Figuren 3 bis 5 abgewandelten Handhabungsvorrichtung, die einen ersten Instrumententräger zur Aufnahme eines ersten Instruments und einen zweiten Instrumententräger zur Aufnahme eines zweiten Instruments aufweist;
- Fig. 7: eine seitliche Teilansicht der Ausgestaltung gemäß Fig. 3 zur Veranschaulichung von Instrumentenantrieben, die mit einem Instrumententräger gekoppelt sind;
- Fig. 8: eine geschnittene perspektivische Teilansicht der Ausgestaltung gemäß Fig. 7 in einer von Fig. 7 abweichenden Orientierung;
- Fig. 9: eine weitere perspektivische Darstellung der Ausgestaltung gemäß Fig. 7 in einer von Fig. 7 abweichenden Orientierung, wobei aus Veranschaulichungsgründen weitere Komponenten ausgeblendet sind;
- Fig. 10: eine gebrochene Teildarstellung der Gestaltung gemäß Fig. 9 in einer Orientierung, die der Orientierung der Ansicht gemäß Fig. 7 entspricht;
- Fig. 11: eine stark vereinfachte Darstellung eines Längsschnitts durch einen Übertragungsabschnitt eines Instrumententrägers, wobei die der Fig. 11 zugrundeliegende Gestaltung der Ausgestaltung gemäß Fig. 10 zumindest ähnlich ist;
- Fig. 12: eine perspektivische vergrößerte Darstellung eines Verteilerabschnitts eines Instrumententrägers, wobei aus Veranschaulichungsgründen diverse Komponenten ausgeblendet sind;
- Fig. 13: eine weiter vergrößerte Ansicht der Ausgestaltung gemäß Fig. 12 in teilweise geschnittenem Zustand in einer gegenüber der Fig. 12 abgewandelten Orientierung;
- Fig. 14: eine beispielhafte perspektivische rückwärtige Teilansicht von Komponenten einer Handhabungsvorrichtung gemäß den Figuren 3 bis 5 zur Veranschaulichung eines Rotationsantriebs;
- Fig. 15: eine vergrößerte perspektivische Darstellung eines Instrumententrägers zur Veranschaulichung eines Zusammenbaus, wobei aus Veranschaulichungszwecken Komponenten ausgeblendet sind;
- Fig. 16: eine Teilschnittdarstellung eines Aufnahmeabschnitts eines Instrumententrägers, an dem ein Instrumentenarm aufgenommen ist;
- Fig. 17: eine perspektivische distale Ansicht eines Aufnahmeabschnitts eines Instrumententrägers zur Veranschaulichung einer Antriebsschnittstelle, wobei aus Veranschaulichungsgründen Komponenten ausgeblendet sind;
- Fig. 18: eine weitere perspektivische Teildarstellung der Gestaltung gemäß Fig. 17, wobei weitere Komponenten ausgeblendet sind;
- Fig. 19: eine proximale Ansicht einer Ausgestaltung eines Übertragungsanschlusses mit einem Mitnahmeprofil;
- Fig. 20: eine perspektivische Darstellung der Gestaltung gemäß Fig. 19 zur Veranschaulichung einer Mitnahmeausnehmung; und
- Fig. 21: eine abgewickelte Darstellung einer kreisförmigen Anordnung einer Mehrzahl von Übertragungsanschlüssen, die zueinander axial versetzt sind und mit Instrumenteneingängen eines Instruments zu koppeln sind.

Zu Erläuterungszwecken ist in zumindest einigen der nachfolgend beschriebenen Figuren ein (kartesisches) Koordinatensystem X-Y-Z dargestellt, das nachfolgend zur Veranschaulichung bestimmter Richtungen und Orientierungen benutzt werden soll. Es versteht sich, dass das Koordinatensystem X-Y-Z lediglich zur Veranschaulichung und Erläuterung dient und nicht dazu, den Gegenstand der Offenbarung einzuschränken. Es versteht sich ferner, dass zur Beschreibung der verschiedenen Ausgestaltungen und Aspekte dieser Offenbarung auch andere Koordinatensysteme mit anderen Orientierungen und Zuordnungen verwendbar sind. Es liegt im Bereich des fachmännischen Handelns, entsprechende (gedankliche) Transformationen vorzunehmen.

Dasselbe trifft nachfolgend auch auf Richtungsangaben und Angaben zur räumlichen Einordnung zu, wie etwa oben, unten, seitlich, vorne, hinten usw. Auch die Verwendung derartiger Begriffe soll nicht in einem einschränkenden Sinne verstanden werden. Sofern im Zusammenhang mit bestimmten Darstellungen und Orientierungen derartige Begriffe verwendet werden, können diese auf die tatsächlich gezeigte Darstellung bezogen sein und demgemäß bei abgewandelten Darstellungen, verbunden mit geänderten Orientierungen und Blickrichtungen, durch entsprechende abgewandelte Zuordnungsbegriffe ersetzt werden.

Nachfolgend wird eine Ansicht, die senkrecht zur X-Richtung orientiert ist, als Seitenansicht bezeichnet. Ferner wird eine Ansicht, die senkrecht zur Z-Richtung orientiert ist, als frontale (oder rückwärtige) Ansicht bezeichnet. Eine Ansicht, die senkrecht zur Y-Achse orientiert ist, wird als Draufsicht oder Ansicht von unten bezeichnet.

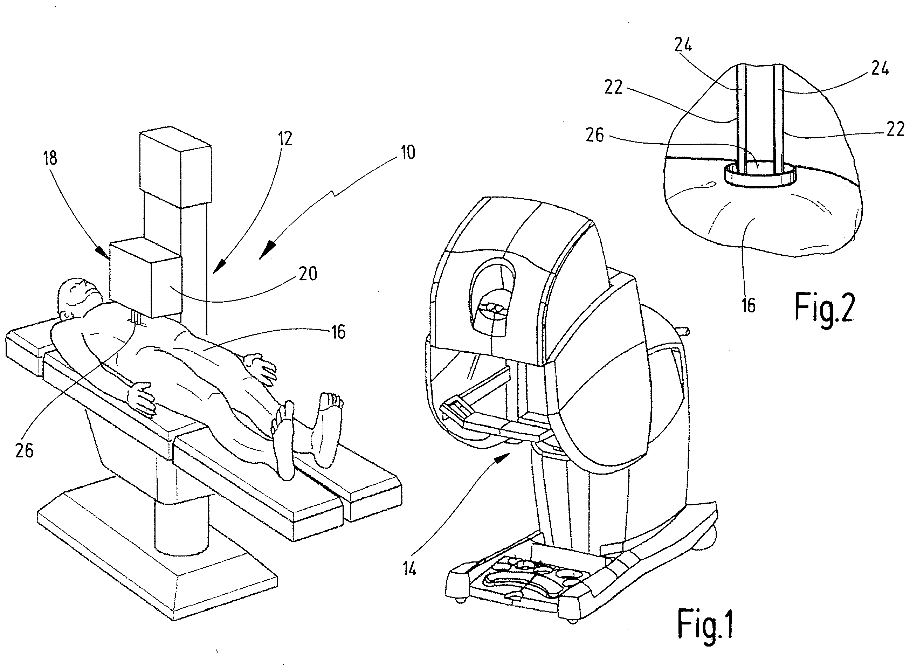

Fig. 1 zeigt eine perspektivische vereinfachte Ansicht einer Operationsumgebung 10, die auch als telemedizinisches Operationssystem bezeichnet werden kann. In der Operationsumgebung 10 ist ein Manipulationssystem 12 vorgesehen, das gewissermaßen einem Operateur oder Chirurgen, dem ein Arbeitsplatz 14 zur Verfügung steht, und einem zu behandelnden Patienten 16 zwischengeschaltet ist. Der Operateur oder Chirurg steuert das Manipulationssystem 12 über den Arbeitsplatz 14 durch geeignete Eingaben. Das Manipulationssystem 12 kann auch als robotischer Manipulator oder als Telemanipulationssystem bezeichnet werden. In der Operationsumgebung 10 muss der Arbeitsplatz 14 nicht unmittelbar in der Nähe des Manipulationssystems 12 oder zu diesem benachbart angeordnet sein. Demgemäß sind auch telemedizinische Anwendungen denkbar.

Das in Fig. 1 lediglich exemplarisch dargestellte Manipulationssystem 12 weist beispielhaft eine Säulenanordnung auf und ist einer Aufnahme (Liege) für den Patienten 16 zugeordnet. Das Manipulationssystem 12 umfasst eine Ansteuerungsplattform 18, die über den Arbeitsplatz 14 ansteuerbar ist. Die Ansteuerungsplattform 18 ist dazu ausgebildet, zumindest ein Instrument 22, insbesondere ein chirurgisches Instrument oder ein diagnostisches Instrument, aufzunehmen und zu steuern. Zu diesem Zweck weist die Ansteuerungsplattform 18 zumindest eine Handhabungsvorrichtung 20 auf. Beispielhafte Ausführungsformen von Handhabungsvorrichtungen 20 werden nachfolgend beschrieben.

Fig. 2 zeigt eine stark vereinfachte Detailansicht der Fig. 1 im Bereich einer Körperöffnung des Patienten 16, durch die ein minimal-invasiver Eingriff erfolgt. Ferner sind in Fig. 2 zwei Instrumente 22 angedeutet, die jeweils einen sich länglich erstreckenden Schaft oder Arm 24 umfassen. Die Körperöffnung, die auch als Eingriffsstelle bezeichnet werden kann, ist in den Figuren 1 und 2 mit 26 bezeichnet. Bei minimal-invasiven Eingriffen wird angestrebt, möglichst nur lokale Eingriffsstellen 26 mit möglichst minimaler Ausdehnung zu nutzen. In den Figuren 1 und 2 ist beispielhaft eine sog. Single-Port-Operation dargestellt, bei der lediglich eine einzige Eingriffsstelle (Körperöffnung) 26 genutzt wird. Durch die eine Eingriffsstelle 26 werden zwei Instrumente 22 in das Körperinnere des Patienten 16 eingeführt. Dies stellt hohe Anforderungen an die Handhabungsvorrichtung 20, da die Instrumente 22 einander unmittelbar benachbart geführt und angetrieben werden. Vorzugsweise sind die Instrumente 22 unabhängig voneinander bewegbar bzw. betätigbar, so dass eine Vielzahl von Antriebsorganen bei der Handhabungsvorrichtung 20 vorgesehen ist.

Die Instrumente 22 selbst können beispielhaft als endoskopische Instrumente, laparoskopische Instrumente, chirurgische Instrumente oder dergleichen ausgeführt sein. In diesem Zusammenhang wird auf die DE 10 2012 211 886 A1 verwiesen, die Ausgestaltungen von Instrumenten 22 mit mehreren Bewegungsfreiheitsgraden bzw. Betätigungsfreiheitsgraden offenbart.

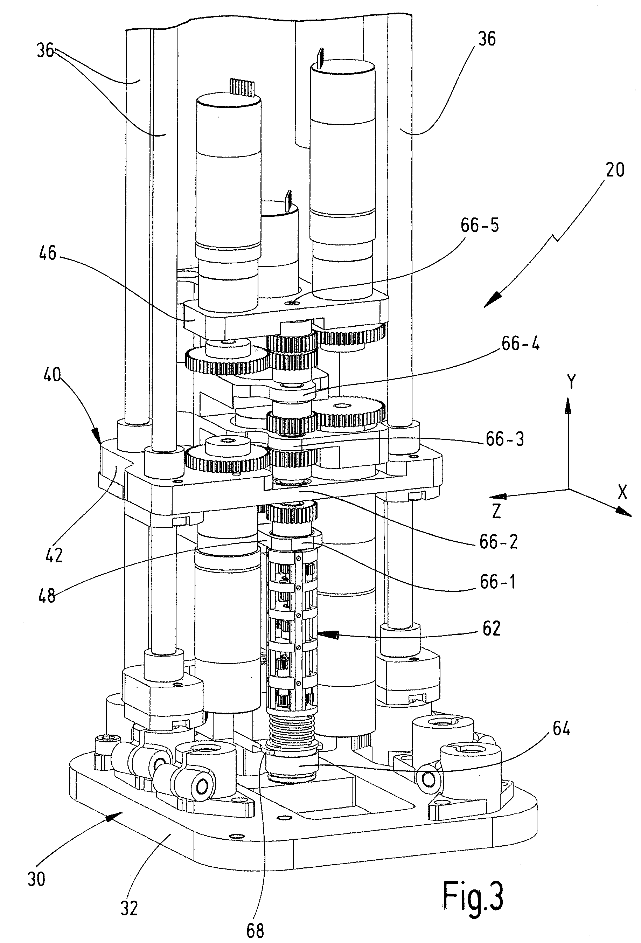

Mit Bezugnahme auf die Figuren 3, 4 und 5 wird eine beispielhafte Ausgestaltung einer Handhabungsvorrichtung 20 für Instrumente 22 veranschaulicht, die Bestandteil des Manipulationssystems 12 und der Operationsumgebung 10 sein kann. Die Handhabungsvorrichtung 20 umfasst ein Gestell 30, das gemäß der in den Figuren 3 bis 5 veranschaulichten Ausgestaltung eine Grundplatte 32 und eine der Grundplatte 32 gegenüberliegende Grundplatte 34 umfasst. Zwischen den Grundplatten 32, 34 erstrecken sich Führungssäulen 36, die eine Längsführung 38 definieren. An der Längsführung 38 ist ein Schlitten 40 verfahrbar aufgenommen.

Die Längsführung 38 erlaubt eine Bewegung oder Verlagerung des Schlittens 40 in einer Längsrichtung (in den Figuren 3 bis 5 parallel zur Y-Richtung). Sofern in dieser Offenbarung von einer Längsrichtung oder Längsbewegung die Rede ist, wird darunter insbesondere die Haupterstreckungsrichtung eines Arms oder Schafts 24 eines an der Handhabungsvorrichtung 20 aufgenommenen Instruments 22 verstanden. Je nach globaler Anordnung und Ausrichtung der Handhabungsvorrichtung 20 kann die Längsrichtung einer (globalen) Vertikalrichtung, Horizontalrichtung, Querrichtung oder einer schräg im Raum orientierten Richtung entsprechen. Gemäß der in Fig. 1 beispielhaft gezeigten Konfiguration einer Operationsumgebung 10 ist die Handhabungsvorrichtung 20 dazu ausgebildet, Instrumente 22 "von oben" an den Patienten 16 heranzuführen. Demgemäß fällt die Längsrichtung (Y-Richtung) mit der Vertikalrichtung zusammen. Dies ist jedoch nicht in einschränkendem Sinne zu verstehen.

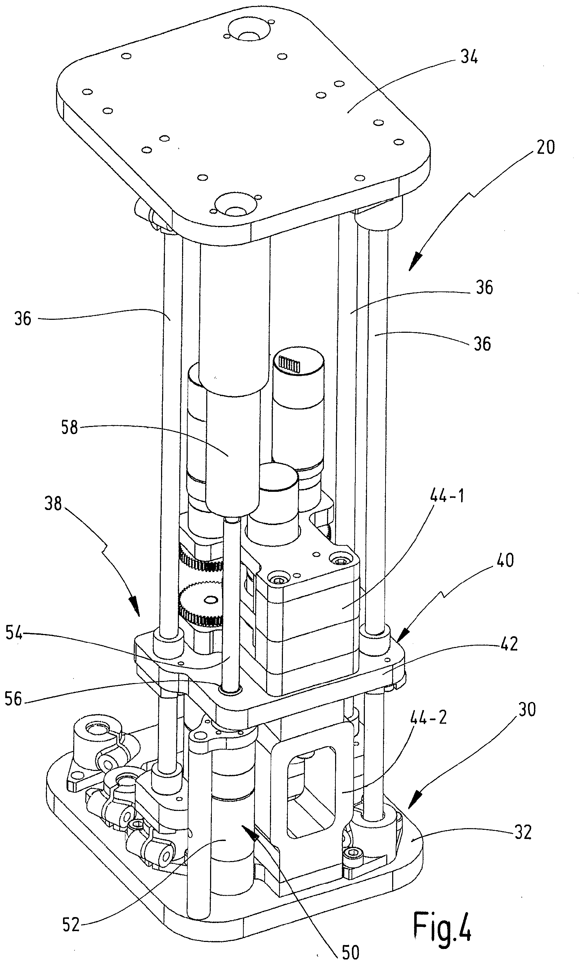

In den Figuren 3 bis 5 ist die Längsführung 38 als Säulenführung ausgestaltet. Andere Führungsprinzipien (Trapezführung, usw.) sind denkbar. Der Schlitten 40 ist über Buchsen an den Führungssäulen 36 relativ zu diesen verfahrbar aufgenommen. Der Schlitten 40 umfasst eine Schlittenbasis 42, an die sich ein Schlittenrahmen 44 anschließt, vergleiche auch Fig. 4, wobei in Fig. 4 ein erster Teil des Schlittenrahmens mit 44-1 und ein zweiter Teil des Schlittenrahmens mit 44-2 bezeichnet, wobei sich die beiden Teile 44-1, 44-2 ausgehend von der Schlittenbasis 42 in entgegengesetzten Richtungen erstrecken. Der Schlitten 40 kann auch als Reiter bezeichnet werden. Der Schlittenrahmen 44 trägt beispielhaft einen ersten Anschlusssteg 46 und einen zweiten Anschlusssteg 48, vgl. auch Fig. 3. Die Anschlussstege 46 und 48 sind in der Längsrichtung (Y-Richtung) von der Schlittenbasis versetzt und dienen zur Aufnahme bzw. zur Führung weiterer Komponenten der Handhabungsvorrichtung 20.

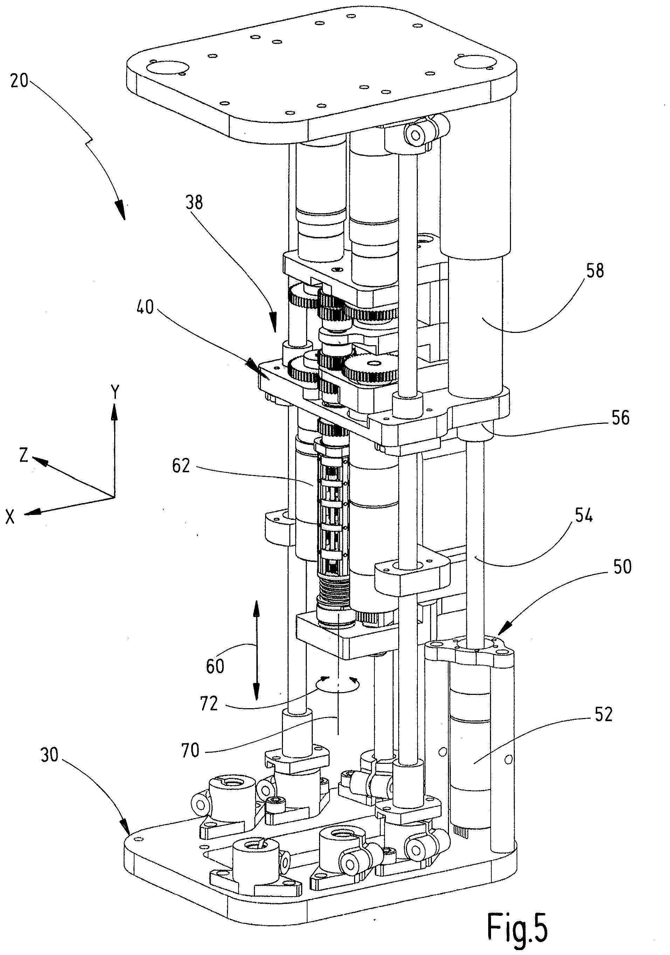

Mit besonderem Verweis auf die Figuren 4 und 5 wird ein Längsantrieb 50 veranschaulicht, der auch als Schlittenantrieb bezeichnet werden kann. Der Längsantrieb 50 ist dazu ausgestaltet, den Schlitten 40 und daran aufgenommene Komponenten in der Längsrichtung Y zu verfahren, vgl. auch einen mit 60 bezeichneten Doppelpfeil in Fig. 5. Der Längsantrieb 50 umfasst einen Motor 52, der eine Spindel 54 antreibt. Die Spindel 54 ist mit einer Spindelmutter 56 gekoppelt, die am Schlitten 40, insbesondere an der Schlittenbasis 42aufgenommen ist. Die Spindelmutter 56 ist verdrehfest am Schlitten 40 aufgenommen und relativ zur bzw. entlang der Spindel 54 verfahrbar. Gemeinsam können die Spindel 54 und die Spindelmutter 56 einen Gewindespindeltrieb bzw. einen Kugelgewindespindeltrieb bilden. Der Motor 52 ist am Gestell 30 aufgenommen, insbesondere an der Grundplatte 32. Der Motor 52 kann die Spindel 54 direkt oder über ein zwischengeschaltetes Getriebe antreiben. Die Spindel 54 ist gestellseitig ferner in einer Spindelführung 58 geführt, die an der (oberen) Grundplatte 34 aufgenommen ist. Gemäß der anhand der Figuren 4 und 5 veranschaulichten Ausgestaltung des Längsantriebs 50 sind der Motor 52, die Spindel 54 und die Spindelführung 58 konzentrisch zueinander in Reihe angeordnet. Diese Ausgestaltung beansprucht einen gewissen axialen Bauraum (in der Längsrichtung).

Der Längsantrieb 50 kann in einer alternativen (nicht dargestellten) Ausgestaltung mittelbar auf die Spindel 54, bspw. die Schraubspindel einwirken. Es ist auch vorstellbar, den Motor 52 parallel zur Spindel 54 und versetzt zu dieser anzuordnen. Zu diesem Zweck kann zwischen dem Motor 52 des Längsantriebs 50 und der Spindel 54 eine Übertragungsstufe (etwa Zahnradstufe) vorgesehen sein. Demgemäß kann der Längsantrieb 50, insbesondere dessen Motor 52, parallel zur Spindel 54 orientiert sein und den gleichen axialen Bauraum wie die Spindel 54 beanspruchen. Dies kann insgesamt zu einem verringerten axialen Bauraumbedarf für den Längsantrieb 50 führen.

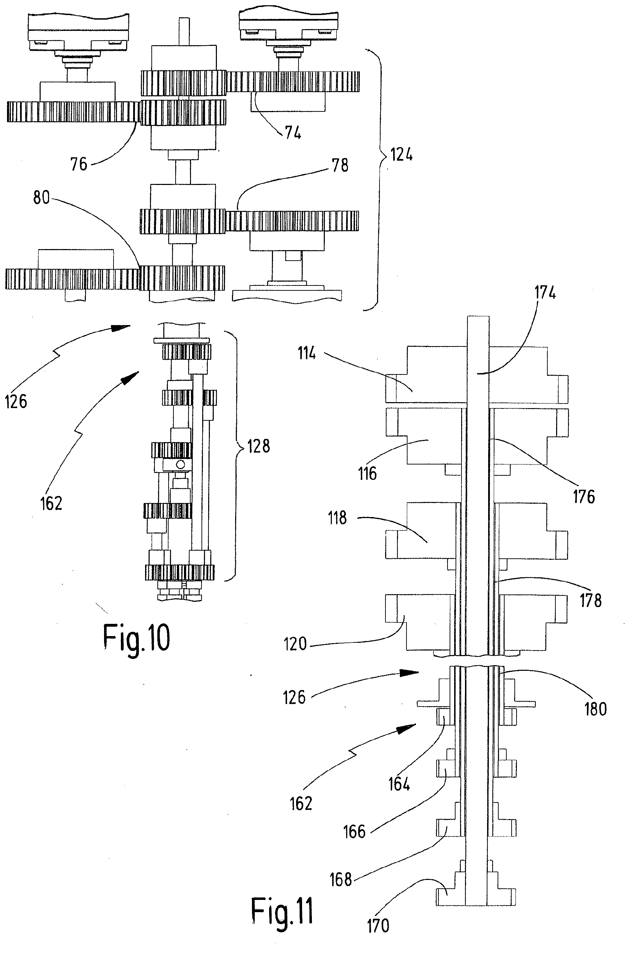

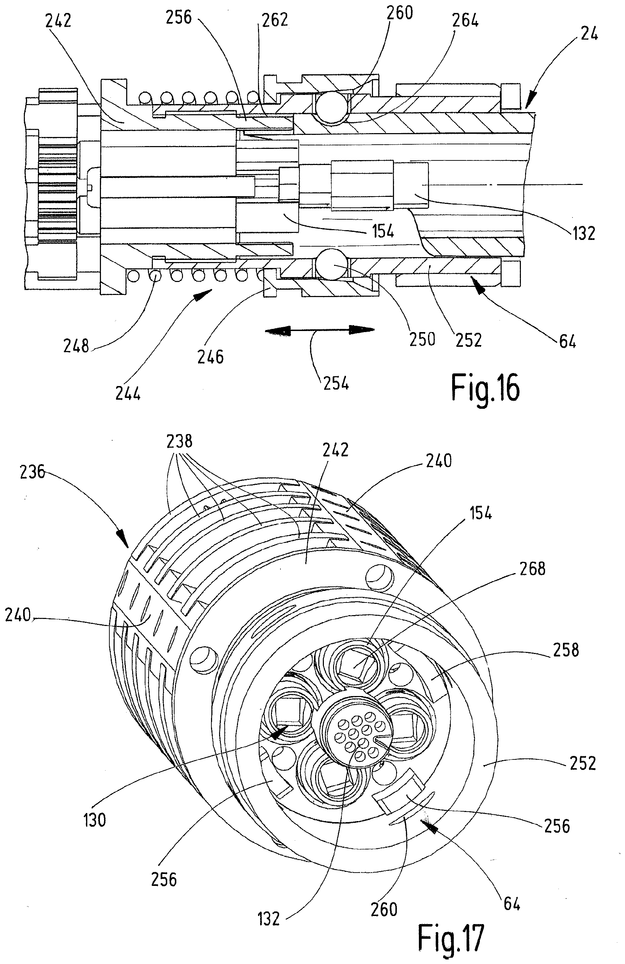

Am Schlitten 40, insbesondere an dessen Schlittenbasis 42, ist ferner ein Instrumententräger 62 aufgenommen, der zur Aufnahme, Führung und Steuerung eines Instruments 22 ausgebildet ist. Hierzu weist der Instrumententräger 62 an seinem distalen Ende einen Aufnahmeabschnitt 64 auf, an dem ein proximales Ende eines Instrumentenarms 24 aufnehmbar ist (vgl. auch Fig. 16). Im gefügten Zustand sind der Instrumentenarm 24 und der Instrumententräger 62 fluchtend zueinander ausgerichtet. Der Instrumententräger 62 ist drehbar bzw. rotierbar am Schlitten 40 aufgenommen. Hierzu ist zumindest ein Lager 66 zwischen dem Schlitten 40 und dem Instrumententräger 62 ausgebildet. In Fig. 3 sind verschiedene Lagerstellen, die unmittelbar oder mittelbar mit dem Instrumententräger 62 gekoppelt sind, mit 66-1, 66-2, 66-3, 66-4 und 66-5 bezeichnet. Eine direkte Lagerung erfolgt an der Lagerstelle 66-1. Eine mittelbare Lagerung des Instrumententrägers 62 erfolgt an den Lagerstellen 66-2 bis 66-5, die Übertragungselemente 174 bis 180 am Schlitten 40 lagern (vgl. auch Fig. 11). Ferner ist dem distalen Ende des Instrumententrägers eine beispielhafte als Führungsgabel ausgestaltete Führung 68 zugeordnet. Die Führung 68 ist am Schlitten 40 aufgenommen und greift in eine Nut am Aufnahmeabschnitt 64 ein.

In Fig. 5 ist eine Längsachse des Instrumententrägers 62 mit 70 angedeutet. Der Instrumententräger 62 ist durch den Längsantrieb 50 gemeinsam mit dem Schlitten 40 in der Längsrichtung verfahrbar (Doppelpfeil 60). Ferner ist der Instrumententräger 62 durch die Lagerung 66 um die Längsachse 70 verdrehbar bzw. rotierbar, vgl. einen gekrümmten Doppelpfeil 72 in Fig. 5.

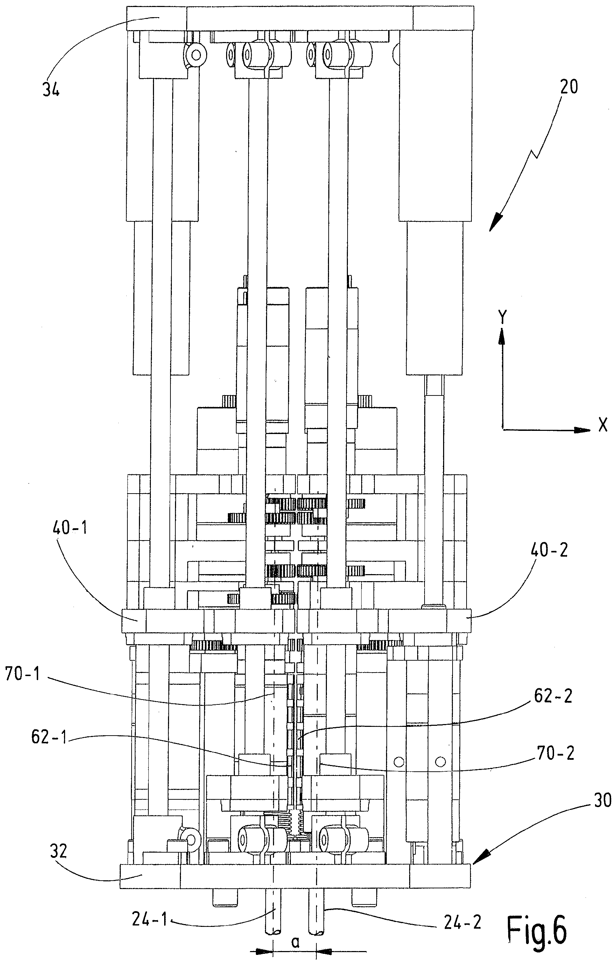

Fig. 6 zeigt eine seitliche Ansicht einer weiteren Ausführungsform einer Handhabungsvorrichtung 20, die in ihrer Grundstruktur grundsätzlich die anhand der Fig. 3, 4 und 5 veranschaulichten Handhabungsvorrichtung 20 entspricht. An der Handhabungsvorrichtung 20 sind jedoch zwei grundsätzlich unabhängig voneinander verfahrbare Schlitten 40-1, 40-2 aufgenommen, die jeweils einen Instrumententräger 62-1, 62-2 tragen, an dem jeweils ein Instrumentenarm 24-1, 24-2 aufgenommen ist. Beispielhaft weist die Handhabungsvorrichtung 20 gemäß Fig. 6 eine gespiegelte Konfiguration bzw. eine um 180° gedrehte Orientierung zwischen den Schlitten 40-1, 40-2 sowie den Instrumententrägern 62-1, 62-2 auf. In den Figuren 3, 4 und 5 wurde aus Veranschaulichungsgründen auf die Darstellung der jeweils zweiten Einheit verzichtet.

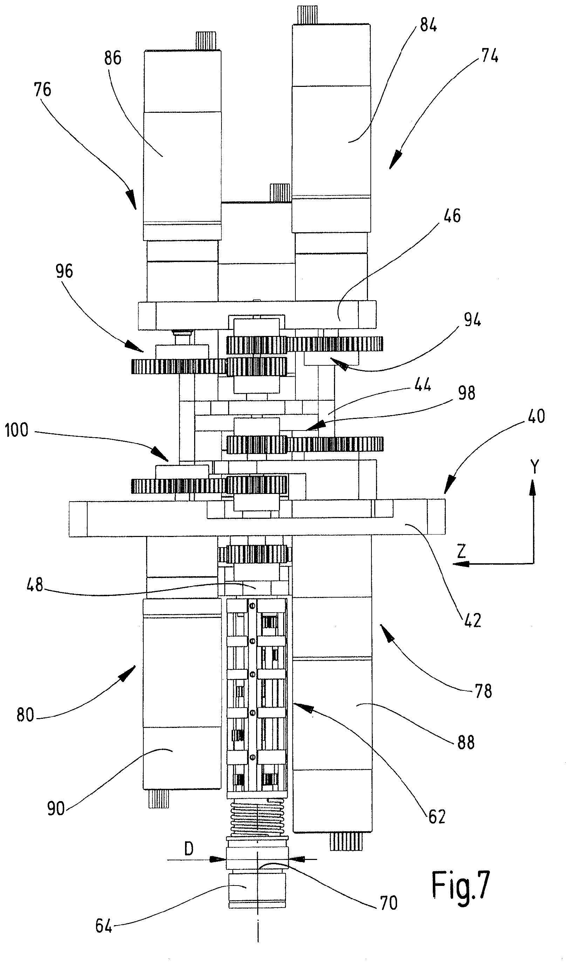

Fig. 6 ist ferner entnehmbar, dass die Instrumententräger 62-1, 62-2 einander unmittelbar benachbart und parallel zueinander angeordnet sind. Dies führt dazu, dass Längsachsen 70-1, 70,2, die durch die Instrumententräger 62-1, 62-2 definiert werden, einen nur geringen Versatzabstand voneinander aufweisen (Abstand in X-Richtung). Dies führt dazu, dass auch die an den Instrumententrägern 62-1, 62-2 aufgenommenen Instrumentenarme 24-1, 24-2 sehr nah aneinander angeordnet werden können. Ein Abstand zwischen den Längsachsen 70-1, 70-2 ist in Fig. 6 mit a bezeichnet. Ein Außendurchmesser der Instrumententräger 62 ist in Fig. 7 mit D bezeichnet. Vorzugsweise erlaubt die Gestaltung der Handhabungsvorrichtung 20 ein äußerst kleines Verhältnis zwischen dem Abstand a und dem Durchmesser D (a/D bzw. a:D). Verschiedene Aspekte der Gestaltung der Handhabungsvorrichtung 20 erlauben die gewünschte räumliche Nähe zwischen den Instrumententrägern 62-1, 62-2 und daran aufgenommenen Instrumenten 22. Dies wird nachfolgend näher erläutert.

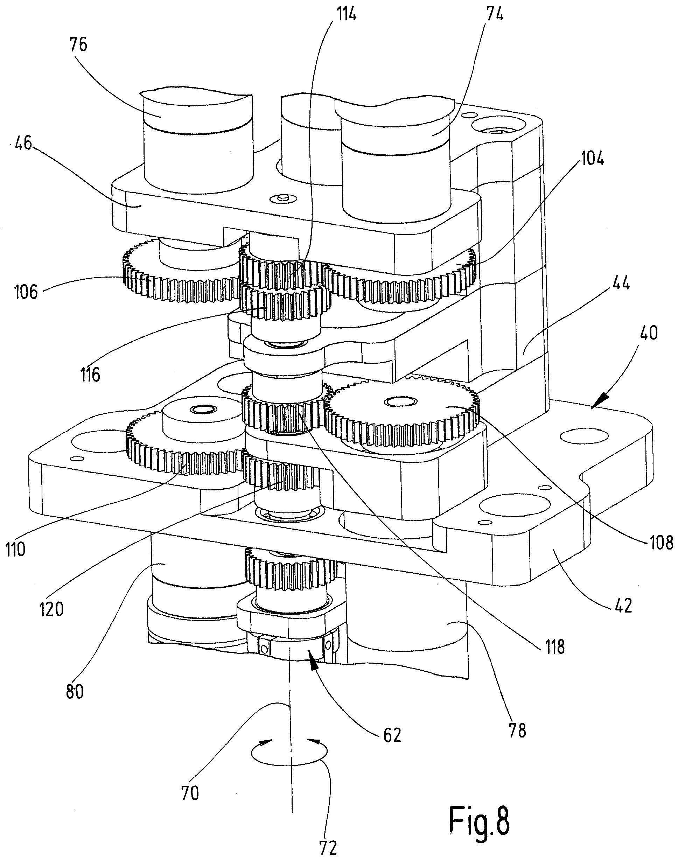

Fig. 7 zeigt zur näheren Veranschaulichung der Ausgestaltung eine frontale Ansicht des Schlittens 40. Fig. 8 zeigt eine korrespondierende vergrößerte perspektivische Teilansicht. Am Schlitten 40 ist in grundsätzlich bereits beschriebener Weise der Instrumententräger 62 aufgenommen. Am Schlitten 40 sind ferner Instrumentenantriebe aufgenommen, beispielhaft ein erster Instrumentenantrieb 74, ein zweiter Instrumentenantrieb 76, ein dritter Instrumentenantrieb 78, und ein vierter Instrumentenantrieb 80. Der erste Instrumentenantrieb 74 weist einen ersten Motor 84 auf. Der zweite Instrumentenantrieb 76 weist einen zweiten Motor 86 auf. Der dritte Instrumentenantrieb 78 weist einen dritten Motor 88 auf. Der vierte Instrumentenantrieb 80 weist einen vierten Motor 90 auf. Die Instrumentenantriebe 74, 76, 78, 80 sind dazu ausgestaltet, Bewegungsfreiheitsgrade bzw. Betätigungsfreiheitsgrade auf Seiten eines angekoppelten Instruments 22 anzusteuern, um Aktivitäten des Instruments 22 zu veranlassen.

Die Instrumentenantriebe 74, 76, 78, 80 sind nicht dazu ausgestaltet, den Instrumententräger 62 selbst anzutreiben. Vielmehr werden Bewegungen, die durch die Instrumentenantriebe 74, 76, 78, 80 erzeugt werden, gewissermaßen durch den Instrumententräger 62 hindurch in das angekoppelte Instrument 22 übertragen. Die Instrumentenantriebe 74, 76, 78, 80 und deren Motoren 84, 86, 88, 90 sind parallel zur Längsachse 70 orientiert. Die Instrumentenantriebe 74 und 76 sind am Anschlusssteg 46 des Schlittens 40 aufgenommen. Die Instrumentenantriebe 78 und 80 sind an der Schlittenbasis 42 des Schlittens 40 aufgenommen. Insgesamt ergibt sich eine etwa H-förmige Konfiguration der Instrumentenantriebe 74, 76, 78, 80, wobei der erste Instrumentenantrieb 74 und der zweite Instrumentenantrieb 76 sowie der dritte Instrumentenantrieb 78 und der vierte Instrumentenantrieb 80 jeweils aufeinander gegenüberliegenden Seiten der Längsachse 70 des Instrumententrägers 62 angeordnet sind. Andere Gestaltungen sind denkbar.

Zur Überleitung der Antriebsbewegung in den Instrumententräger 62 (bzw. durch diesen hindurch) ist der erste Instrumentenantrieb 74 mit einer ersten Eingangsstufe 94 gekoppelt. Der zweite Instrumentenantrieb 76 ist mit einer zweiten Eingangsstufe 96 gekoppelt. Der dritte Instrumentenantrieb 78 ist mit einer dritten Eingangsstufe 98 gekoppelt. Der vierte Instrumentenantrieb 80 ist mit einer vierten Eingangsstufe 100 gekoppelt. Die Eingangsstufen 94, 96, 98, 100 sind an einem proximalen Ende des Instrumententrägers 62 mit diesem gekoppelt. Die Eingangsstufen 94, 96, 98, 100 sind als Zahnradstufen ausgeführt. Die erste Eingangsstufe 94 umfasst ein erstes Ritzel 104 und ein erstes Rad 114. Die zweite Eingangsstufe 96 umfasst ein zweites Ritzel 106 und ein zweites Rad 116. Die dritte Eingangsstufe 98 umfasst ein drittes Ritzel 108 und ein drittes Rad 118. Die vierte Eingangsstufe 100 umfasst ein viertes Ritzel 110 und ein viertes Rad 120.

Es versteht sich, dass vom Umfang der vorliegenden Offenbarung auch Gestaltung von Instrumententrägern 62 umfasst sind, die zwei, drei oder sogar mehr als vier Instrumentenantriebe 74, 76, 78, 80 aufweisen. Durch die Instrumentenantriebe 74, 76, 78, 80 können die diesen zugeordneten Räder 114, 116, 118, 120 um die Längsachse 70 verdreht werden. Dies bewirkt jedoch keine Verdrehung des Instrumententrägers 62 als solches um Längsachse 70.

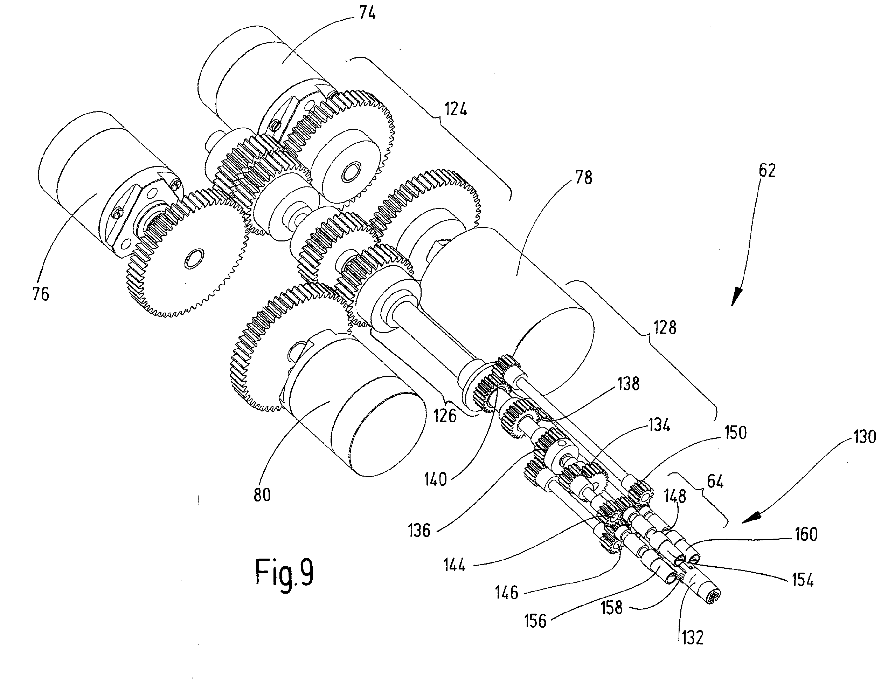

Fig. 9 zeigt eine perspektivische Ansicht der Gestaltung gemäß den Figuren 7 und 8, wobei aus Veranschaulichungszwecken weitere Komponenten ausgeblendet sind. Der Instrumententräger 62 weist verschiedene Abschnitte auf. An seinem proximalen Ende weist der Instrumententräger 62 einen Eingangsabschnitt 124 auf. Im Bereich des Eingangsabschnitts 124 sind die Eingangsstufen 94, 96, 98, 100 angeordnet. An den Eingangsabschnitt 124 schließt sich ein Übertragungsabschnitt 126 an. Der Übertragungsabschnitt 126 kann auch als konzentrischer Übertragungsabschnitt bezeichnet werden. Der Übertragungsabschnitt 126 umfasst eine konzentrische Wellenanordnung (vgl. auch Fig. 11). An den Übertragungsabschnitt 126 schließt sich ein Verteilerabschnitt 128 an, in dem Bewegungen bzw. Momente, die über den Übertragungsabschnitt 126 übertragen werden, abgegriffen werden. An den Verteilerabschnitt 128 schließt sich eine Antriebsschnittstelle 130 an, die dem Aufnahmeabschnitt 64 des Instrumententrägers 62 zugeordnet ist. Ferner ist in Fig. 9 eine Kontaktschnittstelle 132 in einem zentralen Bereich des Aufnahmeabschnitts 64 angedeutet.

Im Eingangsabschnitt 124 werden Bewegungen bzw. Momente von den exzentrisch angeordneten Instrumentenantrieben 74, 76, 78, 80 an eine konzentrische Wellenanordnung im Übertragungsabschnitt 126 übertragen. Im Verteilerabschnitt 128 erfolgt umgekehrt eine Übertragung der Bewegung bzw. Momente von der konzentrischen Wellenanordnung hin zu exzentrisch angeordneten Elementen, nämlich zu Übertragungsanschlüssen 154, 156, 158, 160. Zu diesem Zweck umfasst der Verteilerabschnitt 128 eine erste Übergangsstufe 134, die mit einer ersten Abtriebsstufe 144 gekoppelt ist. Ferner ist eine zweite Übergangsstufe 136 vorgesehen, die mit einer zweiten Abtriebsstufe 146 gekoppelt ist. Daneben ist eine dritte Übergangsstufe 138 vorgesehen, die mit einer dritten Abtriebsstufe 148 gekoppelt ist. Ferner ist eine vierte Übergangsstufe 140 vorgesehen, die mit einer vierten Abtriebsstufe 150 gekoppelt ist. Die erste Abtriebsstufe 144 mündet in den ersten Übertragungsanschluss 154. Die zweite Abtriebsstufe 146 mündet in den zweiten Übertragungsanschluss 156. Die dritte Abtriebsstufe 148 mündet in den dritten Übertragungsanschluss 158. Die vierte Abtriebsstufe 150 mündet in den vierten Übertragungsanschluss 160.

Die Antriebsschnittstelle 130 mit den Übertragungsanschlüssen 154, 156, 158, 160 dient zur Übertragung mechanischer Energie hin zum Instrument 22, insbesondere zur Übertragung von Drehbewegungen bzw. von Drehmomenten. Die Kontaktschnittstelle 132 dient zur Übertragung von elektrischer Energie bzw. zur Übertragung von elektrischen Signalen zum Instrument 22, und umgekehrt.

Insgesamt erlaubt die in Fig. 9 gezeigte Ausgestaltung des Instrumententrägers 62 die Übertragung vier separater Antriebsbewegungen zum das Instrument 22. Diese können auch als Bewegungsfreiheitsgrade für das Instrument 22 bezeichnet werden. Ein erster Übertragungspfad für einen ersten Bewegungsfreiheitsgrad erstreckt sich vom ersten Instrumentenantrieb 74 hin zum ersten Übertragungsanschluss 154. Ein zweiter Pfad für einen zweiten Bewegungsfreiheitsgrad erstreckt sich vom zweiten Instrumentenantrieb 76 hin zum zweiten Übertragungsanschluss 156. Ein dritter Pfad für einen dritten Bewegungsfreiheitsgrad erstreckt sich vom dritten Instrumentenantrieb 78 hin zum dritten Übertragungsanschluss 158. Ein vierter Pfad für einen vierten Bewegungsfreiheitsgrad erstreckt sich vom vierten Instrumentenantrieb 80 hin zum vierten Übertragungsanschluss 160. Die Anordnung stellt vier Bewegungsfreiheitsgrade bereit.

Unter Zwischenschaltung des konzentrischen Übertragungsabschnitts 126 werden Bewegungen von exzentrisch angeordneten Instrumentenantrieben 74, 76, 78, 80 hin zu exzentrisch angeordneten Übertragungsanschlüssen 154, 156, 158, 160 übertragen, und zwar grundsätzlich unabhängig voneinander. Die genutzten Bewegungspfade bzw. Übertragungspfade nutzen im Bereich des Übertragungsabschnitts 126 die gleiche (geometrische) Drehachse.

Die anhand der Fig. 9 veranschaulichte Gestaltung des Instrumententrägers 62 kann ferner abgewandelt werden, wenn die Übergangsstufen 134, 136, 138, 140 direkt in die Übertragungsanschlüsse 154, 156, 158, 160 münden. Gemäß dieser Ausgestaltung sind keine (weiteren) Abtriebsstufen 144, 146, 148, 150 erforderlich.

Mit Verweis auf die Figuren 10 und 11, die jeweils gebrochene, seitliche Teilansichten der Anordnung gemäß Fig. 9 zeigen, wird die Gestaltung des Übertragungsabschnitts 126 und der daran angrenzenden Abschnitte (Eingangsabschnitt 124 und Verteilerabschnitt 128) näher veranschaulicht. Fig. 11 zeigt einen vereinfachten, schematischen Längsschnitt. Im Eingangsabschnitt 124 erfolgt eine Einleitung der Antriebsbewegungen in den Übertragungsabschnitt 126. Im Verteilerabschnitt 128 erfolgt ein Abtrieb der durch den Übertragungsabschnitt 126 übertragenen Bewegungen. Im Übertragungsabschnitt 126 ist ein Wellenverbund ausgebildet, der auch als konzentrische Wellenanordnung 162 bezeichnet werden kann. Die Wellenanordnung 162 umfasst Übertragungselemente 174, 176, 178, 180, die eingangsseitige Räder 114, 116, 118, 120 mit ausgangsseitigen (distalen) Abtriebsritzeln 164, 166, 168, 170 verbinden.

Ein erstes Übertragungselement 174 ist beispielhaft als (zentrale) Welle ausgestaltet. Ein zweites Übertragungselement 176 ist als Rohr oder Hohlwelle gestaltet und umschließt das erste Übertragungselement 174. Ein drittes Übertragungselement 178 ist als Rohr oder Hohlwelle gestaltet und umschließt das zweite Übertragungselement 176. Ein viertes Übertragungselement 180 ist als Rohr oder Hohlwelle gestaltet und umschließt das dritte Übertragungselement 178. Das erste Übertragungselement 174 verbindet das erste Rad 114 mit dem ersten Abtriebsritzel 164. Das zweite Übertragungselement 176 verbindet das zweite Rad 116 mit dem zweiten Abtriebsritzel 166. Das dritte Übertragungselement 178 verbindet das dritte Rad 118 mit dem dritten Abtriebsritzel 168. Das vierte Übertragungselement 180 verbindet das vierte Rad 120 mit dem vierten Abtriebsritzel 170. Demgemäß verbinden die Übertragungselemente 174, 176, 178, 180 jeweils die ihnen zugeordnete Eingangsstufe 94, 96, 98, 100 mit der zugehörigen abtriebsseitigen Übergangsstufe 134, 136, 138, 140.

Prinzipbedingt weist das erste Übertragungselement 174 die größte (axiale) Längserstreckung auf. Es folgen mit absteigender Reihenfolge das zweite Übertragungselement 176, das dritte Übertragungselement 178 und das vierte Übertragungselement 180. Die Übertragungselemente 174, 176, 178, 180 erlauben die Übertragung von Steuerbewegungen bzw. Antriebsmomenten hin zum Instrument 22 durch den Instrumententräger 62 hindurch, obwohl dieser verdrehbar am Schlitten 40 aufgenommen ist.

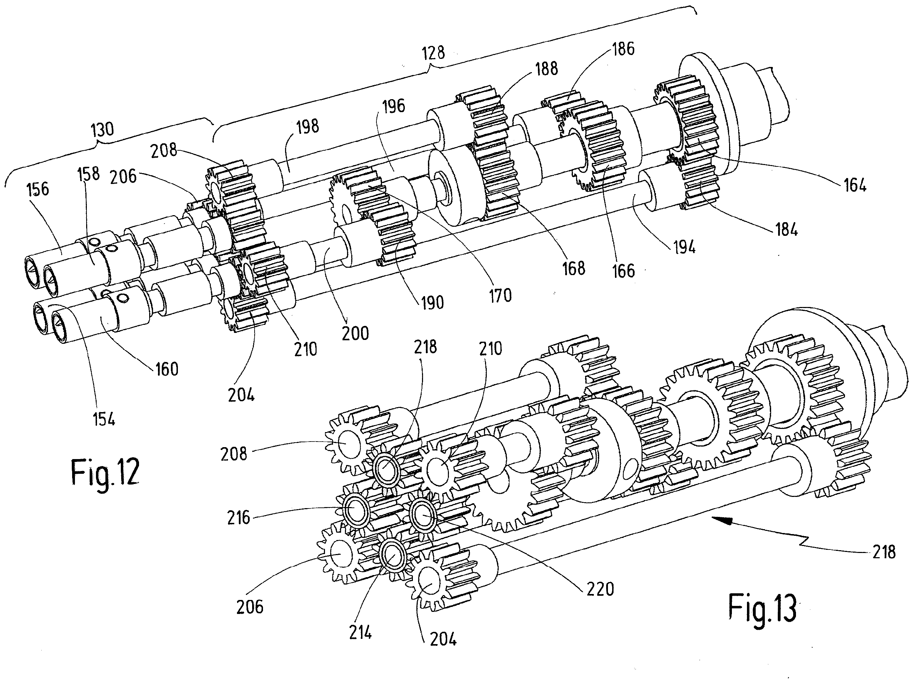

Mit Verweis auf die Figuren 12 und 13 wird der Verteilerabschnitt 128 des Instrumententrägers 62 näher veranschaulicht. Im Verteilerabschnitt 128 werden die konzentrisch übertragenen Bewegungen für die vier Freiheitsgrade durch die Übergangsstufen 134, 136, 138, 140 entlang exzentrisch zur Längsachse versetzter Elemente hin zu den Abtriebsstufen 144, 146, 148, 150 übertragen.

Die erste Übergangsstufe 134 umfasst ein erstes Umfangsrad 184, das mit einer ersten Versatzwelle 194 gekoppelt ist, an deren distalem Ende ein erstes Versatzritzel 204 aufgenommen ist, das der ersten Abtriebsstufe 144 zugeordnet ist. Die zweite Übergangsstufe 136 umfasst ein zweites Umfangsrad 186, das mit einer zweiten Versatzwelle 196 gekoppelt ist, die in ein zweites Versatzritzel 206 mündet, das der zweiten Abtriebsstufe 146 zugeordnet ist. Die dritte Übergangsstufe 138 umfasst ein drittes Umfangsrad 188, das mit einer dritten Versatzwelle 198 gekoppelt ist, die in ein drittes Versatzritzel 208 mündet, das der dritten Abtriebsstufe 148 zugeordnet ist. Die vierte Übergangsstufe 140 umfasst ein viertes Umfangsrad 190, das mit einer vierten Versatzwelle 200 gekoppelt ist, die in ein viertes Versatzritzel 210 mündet, das der vierten Abtriebsstufe 150 zugeordnet ist.

Mit Verweis auf Fig. 13 wird ersichtlich, dass die erste Abtriebsstufe 144 ferner ein erstes Versatzrad 214 aufweist, das mit dem ersten Übertragungsanschluss 154 gekoppelt ist. Die zweite Abtriebsstufe 146 umfasst ferner ein zweites Versatzrad 216, das mit dem zweiten Übertragungsanschluss 156 gekoppelt ist. Die dritte Abtriebsstufe 148 umfasst ferner ein drittes Versatzrad 218, das mit dem dritten Übertragungsanschluss 158 gekoppelt ist. Die vierte Abtriebsstufe 150 umfasst ferner ein viertes Versatzrad 220, das mit dem vierten Übertragungsanschluss 160 gekoppelt ist.

Mit anderen Worten erfolgt bei den Abtriebsstufen 144, 146, 148, 150 ein erneuter exzentrischer Versatz, um die gewünschten Positionen der Übertragungsanschlüsse 154, 156, 158, 160 zu erreichen. Insgesamt sind die Übertragungsanschlüsse 154, 156, 158, 160 kreisförmig um die Längsachse 70 des Instrumententrägers 62 verteilt. Es versteht sich, dass in zumindest einigen Ausgestaltungen auf die letzte Stufe (Abtriebsstufe 144, 146, 148, 150) verzichtet werden kann, wenn die Übertragungsanschlüsse 154, 156, 158, 160 direkt an die Versatzwellen 194, 196, 198, 200 ankoppeln.

Die vorstehend beschriebene Ausgestaltung des Instrumententrägers 62 und der Instrumentenantriebe 74, 76, 78, 80 hat den besonderen Vorteil, dass der Instrumententräger 62 um seine Längsachse verdreht oder rotiert werden kann und während dieser Rotationsbewegung die Motoren 84, 86, 88, 90 der Instrumentenantriebe 74, 76, 78, 80 nicht mitbewegt bzw. mitverschwenkt werden müssen. Dies kann insgesamt den Bauraum der Handhabungsvorrichtung 20 deutlich minimieren und erlaubt beispielhaft eine benachbarte Anordnung zweier Instrumententräger 62-1, 62-2 nahe beieinander, vgl. auch Fig. 6.

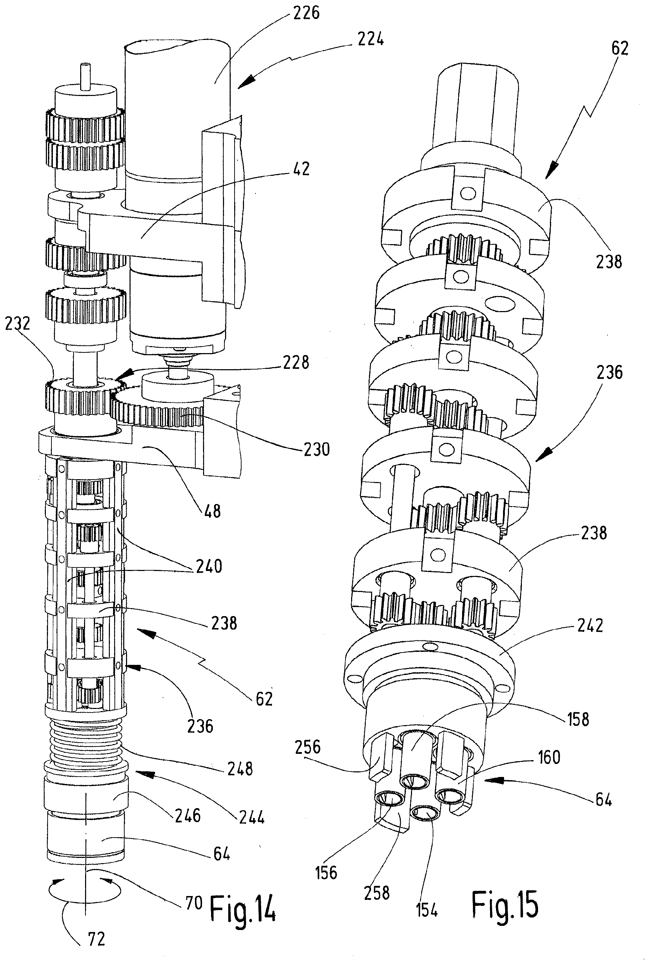

Mit Bezug auf Fig. 14 wird ein Rotationsantrieb 224 für den Instrumententräger 62 näher veranschaulicht. Der Rotationsantrieb 224 erlaubt eine Verdrehung des Instrumententrägers 62 um seine Längsachse 70, vgl. den gekrümmten Doppelpfeil 72 in Fig. 14. Der Rotationsantrieb 224 umfasst einen Motor 226, der am Schlitten 40 aufgenommen ist, insbesondere an der Schlittenbasis 42. Der Motor 226 ist über eine Zahnradstufe 228, die ein Ritzel 230 und ein Rad 232 umfasst, mit dem Instrumententräger 62 gekoppelt. Das Rad 232 ist drehfest mit dem Aufnahmeabschnitt 64 bzw. der Antriebsschnittstelle 130 verbunden, um einen aufgenommenen Instrumentenarm 24 gemeinsam mit dem Instrumententräger 62 um die Längsachse 70 verdrehen zu können.

Während der Bewegung des Instrumententrägers 62 verbleiben die Motoren 84, 86, 88, 90 der Instrumentenantriebe 74, 76, 78, 80 an ihren angestammten Positionen am Schlitten 40. Dies kann unter bestimmten Umständen zu unerwünschten parasitären Abtriebsbewegungen bei den Übertragungsanschlüssen 154, 156, 158, 160 führen. Zur Vermeidung dieser Bewegungen können die Instrumentenantriebe 74, 76, 78, 80 geeignet angesteuert werden, um eine definierte Gegenbewegung zum Ausgleich der parasitären Bewegung zu bewirken.

Dies kann beispielsweise eine Erfassung oder Berechnung der tatsächlichen (globalen) Drehbewegung des Instrumententrägers 62 gegenüber dem Schlitten 40 bzw. dem Gestell 30 umfassen, auf deren Basis eine erforderliche Gegenbewegung der Instrumentenantriebe 74, 76, 78, 80 bestimmt wird, um aus Sicht der Übertragungsanschlüsse 154, 156, 158, 160 die (globale) Drehbewegung des Instrumententrägers 62 zu kompensieren. Die Motoren 84, 86, 88, 90 der Instrumentenantriebe 74, 76, 78, 80 können entsprechend angesteuert werden, so dass das aufgenommene Instrument trotz der (globalen) Drehbewegung um die Längsachse 70 in einem stabilen internen Zustand verbleibt.

Eine weitere Möglichkeit zur Vermeidung bzw. Kompensation von parasitären Bewegungen besteht darin, die Instrumentenantriebe 74, 76, 78, 80 bzw. die Eingangsstufen 94, 96, 98, 100 von den Elementen bzw. Komponenten abzukoppeln, die sich an das distale Ende der konzentrischen Wellenanordnung 162 anschließen. Zu diesem Zweck kann bspw. eine Selbsthemmung im Antriebsstrang genutzt werden, die bewirkt, dass bei einer Verdrehung des Instrumententrägers 62 gewissermaßen ein Mitbewegen der Eingangsstufen 94, 96, 98, 100 und ggf. der Motoren 84, 86, 88, 90 erfolgt. Es ist auch vorstellbar, die Motoren 84, 86, 88, 90 gezielt zu entkoppeln.

Eine weitere, alternative Möglichkeit zur Kompensation der parasitären Bewegungen besteht darin, die beteiligten Zahnradstufen derart auszulegen, dass zwischen dem proximalen Eingang und dem distalen Ausgang des Instrumententrägers eine Richtungsumkehr aber betragsmäßig (winkelmäßig) gleiche Übertragung der durch die Instrumentenantriebe 74, 76, 78, 80 bei den Übertragungsanschlüssen 154, 156, 158, 160 bewirkten Drehbewegung erfolgt.

Zwischen dem Rad 232 und dem Aufnahmeabschnitt 64 umfasst der Instrumententräger 62 ein Rotationsgestell 236, das auch als Käfig bezeichnet werden kann. Das Rotationsgestell 236 bewirkt eine drehsteife Verbindung zwischen dem Rad 232 und dem Aufnahmeabschnitt 64 zur Mitnahme des Instrumentenarms 24. Am Rotationsgestell 236 ist ferner der Verteilerabschnitt 128 und, zumindest abschnittsweise, die Antriebsschnittstelle 130 aufgenommen. Zur näheren Gestaltung des Rotationsgestells 236 wird ergänzend auf Fig. 15 verwiesen. Gemäß der anhand der Figuren 14 und 15 veranschaulichten Ausgestaltung ist das Rotationsgestell 236 käfigartig gestaltet und weist eine Anordnung axial zueinander versetzter Lagerplatten 238 auf, die etwa scheibenartig gestaltet sind. In den Lagerplatten 238 sind Ausnehmungen vorgesehen, um die an der Bewegungsübertragung bzw. Kraftübertragung beteiligten Elemente aufnehmen bzw. lagern zu können. Die Lagerplatten 238 des Rotationsgestells 236 sind über eine Mehrzahl von Seitenstegen 240 drehfest miteinander verbunden. Die Seitenstege 240 sind in umfangsseitigen Ausnehmungen der Lagerplatten 238 aufgenommen. Gemeinsam bilden die Lagerplatten 238 und die Seitenstege 240 einen Zylinderkäfig. Am distalen Ende des Rotationsgestells 236 ist ein Abtriebsflansch 242 mit diesem gekoppelt. Der Abtriebsflansch 242 ist zur Drehmitnahme des aufgenommenen Instrumentenarms 24 ausgebildet.

Ferner ist am Abtriebsflansch 242 eine Rastaufnahme 244 aufgenommen. Die Rastaufnahme 244 umfasst eine axial verschiebbare Rasthülse 246, die durch eine Rastfeder 248 axial vorgespannt wird, vgl. auch die Teilschnittdarstellung des Aufnahmeabschnitts 64 in Fig. 16.

Die Rastaufnahme 244 umfasst ferner Rastelemente in Form von Rastkugeln 250, die radial einrücken oder ausrücken können. Die Rastkugeln 250 sind in Ausnehmungen einer Aufnahmebuchse 252 aufgenommen. Dies umfasst beispielhaft eine Aufnahme in einem Kugelsitz 260 in Form einer Umfangsbohrung an der Aufnahmebuchse 252, vgl. auch Fig. 17. Die Aufnahmebuchse 252 ist mit dem Abtriebsflansch 242 gekoppelt. Eine Bewegungsrichtung der Rasthülse 246 ist in Fig. 16 durch einen mit 254 bezeichneten Doppelpfeil angedeutet. Die Rasthülse 246 weist innenseitig eine konische Verjüngung auf, so dass die Rastkugeln 250 bei einer Bewegung der Rasthülse 246 hin zum proximalen Ende des Instrumententrägers 62 die Rastkugeln 250 radial ausrücken können.

In Fig. 16 ist ferner der Instrumentenarm 24 eines aufgenommenen Instruments 22 teilweise angedeutet. Der Instrumentenarm 24 weist einen rohrförmigen Endabschnitt auf, in dem zumindest eine Rastausnehmung 264 in Form einer kugelförmigen Vertiefung oder einer umlaufenden Nut ausgebildet ist. Die Rastkugeln 250 können im Rastzustand in die Rastausnehmungen 264 am Instrumentenarm 24 einrücken. In diesem Zustand werden die Rastkugeln 250 durch die konische Innenfläche der Rasthülse 246 gehalten. Vorzugsweise ist die Steigung bzw. Verjüngung der konischen Innenfläche derart ausgelegt, dass im eingerasteten Zustand Selbsthemmung zwischen der Rasthülse 246 und den Rastkugeln 250 auftritt. Auf diese Weise ist die Verbindung zwischen dem Instrumententräger 62 und dem Instrumentenarm 24 kraftschlüssig bzw. reibschlüssig gesichert. Zusätzlich drückt die Rastfeder 248 die Rasthülse 246 in Richtung ihrer Raststellung. Zum Lösen der Rastverbindungen muss die Rasthülse 246 bewusst gegen die Kraft der Rastfeder 248 in Richtung des proximalen Endes des Instrumententrägers 62 bewegt werden, so dass die Rastkugeln 250 aus den Rastausnehmungen 264 ausrücken können. Dann kann der Instrumentenarm 24 vom Aufnahmeabschnitt 64 gelöst werden. Die Rasthülse 246 weist ferner eine Nut auf, in die eine als Führungsgabel gestaltete Führung 68 eingreifen kann, vgl. auch Fig. 3. Über die Nut kann die Rasthülse 246 gesichert oder betätigt werden.

In den Figuren 15, 16 sowie in Fig. 17 sind ferner Mitnehmer 256 angedeutet, die am Abtriebsflansch 242 aufgenommen sind. Die Mitnehmer 256 sind zur Drehmitnahme des angekoppelten Instruments 22 ausgebildet. Neben den Mitnehmern 256 ist ferner ein Drehlagesicherungselement 258 vorgesehen. Bei dem Drehlagesicherungselement 258 kann es sich beispielhaft um einen vergrößerten Mitnehmer handeln. Das Drehlagesicherungselement 258 stellt sicher, dass der Instrumentenarm 24 nur in einer bestimmten Vorzugsorientierung relativ zum Instrumententräger 62 aufgenommen werden kann. Die Mitnehmer 256 bzw. das Drehlagesicherungselement 258 greifen in korrespondierende Mitnahmeausschnitte 262 am Instrumentenarm 24, vgl. auch Fig. 16. Der Fig. 17 ist ferner eine beispielhafte Ausgestaltung der Kontaktschnittstelle 132 entnehmbar. Die Kontaktschnittstelle 132 stellt eine Mehrzahl elektrischer Anschlüsse bzw. Kontakte bereit, über die elektrische Energie bzw. elektrische Signale übertragen werden können. Beispielhaft ist die Kontaktschnittstelle 132 in einem Zentrum des Aufnahmeabschnitts 64 angeordnet und kann durch ein entsprechendes Gegenelement (Stecker bzw. Buchse) auf Seiten des Instruments 22 kontaktiert werden.

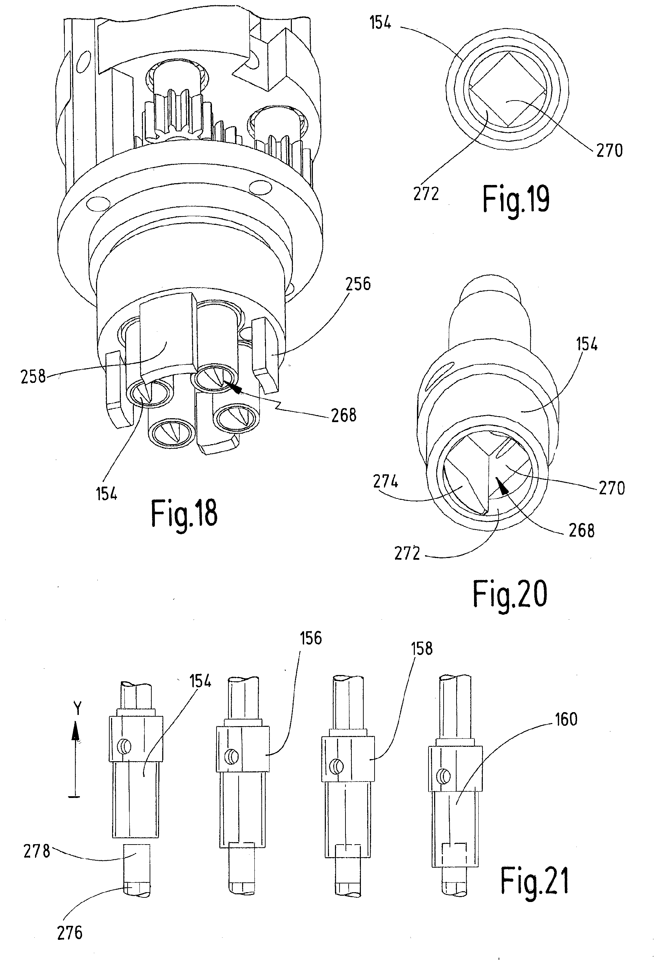

Die Antriebsschnittstelle 130 weist bei den Übertragungsanschlüssen 154, 156, 158, 160 jeweils ein Mitnahmeprofil 268 auf, das zur Mitnahme eines Gegenprofils 278 bei instrumentenseitigen Instrumenteneingängen 276 ausgebildet ist, vgl. insbesondere Fig. 17 bis Fig. 21. Das Mitnahmeprofil 268 umfasst beispielhaft eine Mitnahmeausnehmung 270. Das Mitnahmeprofil 268 ist beispielhaft als Vierkantprofil gestaltet. Andere Ausgestaltungen von Mitnahmeprofilen sind denkbar. Zur Vereinfachung des Fügevorgangs zwischen den Übertragungsanschlüssen 154, 156, 158, 160 und den Instrumenteneingängen 276 ist beim Mitnahmeprofil 268 ferner eine Ausrichtkontur 272 vorgesehen, vgl. Fig. 19 und Fig. 20. Die Ausrichtkontur 272 umfasst Schrägflächen 274, die eine radiale Ausrichtung aber auch eine Drehlagenausrichtung zwischen den Übertragungsanschlüssen 154, 156, 158, 160 und den diesen zugeordneten Instrumenteneingängen 276 (angedeutet in Fig. 21) ermöglichen.

Beispielhaft weisen die Instrumenteneingänge 276 auf Seiten des Instruments 22 Gegenprofile 278 auf, die dazu ausgestaltet sind, in die Mitnahmeprofile 268 bzw. deren Mitnahmeausnehmungen 270 einzurücken. Die Ausrichtkontur 272 mit den Schrägflächen 274 kann dazu beitragen, eine gewünschte Drehlagenorientierung zwischen den Elementen herbeizuführen. Auch ein radialer Versatz zwischen den Koppelelementen kann durch eine entsprechende Einführschräge oder Einführverjüngung ausgeglichen werden.

Fig. 21 veranschaulicht anhand einer beispielhaften Darstellung einer Abwicklung einer eigentlich kreisförmigen Anordnung von Übertragungsanschlüssen 154, 156, 158, 160 eine weitere Ausgestaltung, die das Fügen des Instruments 22 mit dem Instrumententräger 62 vereinfacht. Gemäß dieser beispielhaften Ausgestaltung sind die einzelnen Übertragungsanschlüsse 154, 156, 158, 160 jeweils ein Stück weit axial voneinander versetzt, so dass die Instrumenteneingänge 276 auf Seiten des Instruments 22 nicht gleichzeitig, sondern nacheinander in diese einrücken. Eine umgekehrte Gestaltung mit axial versetzten Instrumenteneingängen 276 ist denkbar.

Auf diese Weise werden Zustände vermieden, bei denen mehrere der Kopplungspaare aus Übertragungsanschluss und Instrumenteneingang nicht hinreichend genau in ihrer gewünschten Orientierung vorliegen. Eine weitere Erleichterung des Fügevorgangs kann durch ein gezieltes Hin- und Herschwenken der Übertragungsanschlüsse 154, 156, 158, 160 durch die ihnen zugeordneten Instrumentenantriebe 74, 76, 78, 80 bewirkt werden. Auf diese Weise können sich die Übertragungsanschlüsse 154, 156, 158, 160 und die Instrumenteneingänge 276 "finden" und ineinander einrücken. Vorzugsweise werden im Rahmen dieses Fügevorgangs (auch als Jiggeln bezeichnet) die Übertragungsanschlüsse 154, 156, 158, 160 mit unterschiedlichen Rotationsgeschwindigkeiten angetrieben, so dass eine Vielzahl von Relativlagen vorliegt, wodurch die Wahrscheinlichkeit erhöht wird, dass sämtliche Paarungen in der gewünschten Relativorientierung vorliegen.

Gemäß einem weiteren Ausführungsbeispiel der Handhabungsvorrichtung 20 sind die Übertragungsanschlüsse 154, 156, 158, 160 um die Längsachse 70 verteilt und exzentrisch zur Längsachse 70 am Instrumententräger 62 aufgenommen. Vorzugsweise definieren die Übertragungsanschlüsse 154, 156, 158, 160 gemeinsam einen Umfangskreis um die Längsachse 70.

Gemäß einem weiteren Ausführungsbeispiel der Handhabungsvorrichtung 20 ist das zumindest eine Übertragungselement 174, 176, 178, 180, vorzugsweise jedes der Übertragungselemente 174, 176, 178, 180 im Übertragungsabschnitt 126, schaftartig oder rohrartig gestaltet und mit einer eingangsseitigen, proximalen Zahnradstufe 94, 96, 98, 100 und einer ausgangsseitigen, distalen Zahnradstufe 144, 146, 148, 150 gekoppelt.

Gemäß einem weiteren Ausführungsbeispiel der Handhabungsvorrichtung 20 weist der Aufnahmeabschnitt 64 des zumindest einen Instrumententrägers 62 eine Kontaktschnittstelle 132 zur Übertragung elektrischer Energie oder elektrischer Signale auf, wobei die Kontaktschnittstelle 132 vorzugsweise in einem Zentrum des Aufnahmeabschnitts 64 angeordnet ist.

## Patentansprüche

1. Handhabungsvorrichtung (20) zur Aufnahme und Handhabung von Instrumenten (22) für minimal-invasive Eingriffe, mit einem Gestell (30) und zumindest einem Instrumententräger (62) mit einem Aufnahmeabschnitt (64) zur Aufnahme eines Instrumentenarms (24),
- wobei der Instrumententräger (62) beweglich am Gestell (30) aufgenommen und mit zumindest einem Antrieb (50, 224) zu dessen Bewegung koppelbar ist, wobei der Instrumententräger (62) zumindest abschnittweise um seine Längsachse (70) relativ zum Gestell (30) rotierbar ist,
- wobei der Aufnahmeabschnitt (64) eine Antriebsschnittstelle (130) zur Übertragung mechanischer Energie zum Instrumentenarm (24) aufweist,
- wobei die Antriebsschnittstelle (130) zumindest einen Übertragungsanschluss (154, 156, 158, 160) zur Bewegungsübertragung, insbesondere zur Momentenübertragung, umfasst,
- wobei dem Übertragungsanschluss (154, 156, 158, 160) ein Instrumentenantrieb (74, 76, 78, 80) zugeordnet ist,
- wobei der Instrumententräger (62) zumindest abschnittsweise entlang seiner Längserstreckung einen Übertragungsabschnitt (126) aufweist, in dem zumindest ein Übertragungselement (174, 176, 178, 180) angeordnet ist, das eine Bewegungsübertragung zwischen dem Instrumentenantrieb (74, 76, 78, 80) und dem Übertragungsanschluss (154, 156, 158, 160) erlaubt,
- wobei das zumindest eine Übertragungselement (174, 176, 178, 180) konzentrisch zur Längsachse (70) des Instrumententrägers (62) ausgerichtet ist, und
- wobei der Übertragungsanschluss (154, 156, 158, 160) exzentrisch zur Längsachse (70) angeordnet ist, so dass der Übertragungsanschluss (154, 156, 158, 160) während der Rotation des Instrumententrägers (62) um dessen Längsachse (70) verschwenkt wird.

2. Handhabungsvorrichtung (20) nach Anspruch 1, wobei die Antriebsschnittstelle (130) des zumindest einen Instrumententrägers (62) zumindest zwei, vorzugweise zumindest drei oder vier, Übertragungsanschlüsse (154, 156, 158, 160) aufweist, von denen zumindest zwei von der Längsachse (70) radialversetzt und voneinander beabstandet angeordnet sind, wobei im Übertragungsabschnitt (126) zumindest zwei, vorzugweise zumindest drei oder vier, Übertragungselemente (174, 176, 178, 180) angeordnet sind, die den zumindest zwei Übertragungsanschlüssen (154, 156, 158, 160) zugordnet sind, wobei zumindest zwei, vorzugweise zumindest drei oder vier, Instrumentenantriebe (74, 76, 78, 80) vorgesehen sind,
- wobei die Handhabungsvorrichtung (20) einen ersten Bewegungsfreiheitsgrad für ein aufgenommenes Instrument (22) bereitstellt, dem ein erster Instrumentenantrieb (74), ein erstes Übertragungselement (174) und ein erster Übertragungsanschluss (154) zugeordnet sind, die zur Drehübertragung miteinander gekoppelt sind, insbesondere durch Zahnradstufen,
- wobei die Handhabungsvorrichtung (20) einen zweiten Bewegungsfreiheitsgrad für ein aufgenommenes Instrument (22) bereitstellt, dem ein zweiter Instrumentenantrieb (76), ein zweites Übertragungselement (176) und ein zweiter Übertragungsanschluss (156) zugeordnet sind, die zur Drehübertragung miteinander gekoppelt sind, insbesondere durch Zahnradstufen, und
- wobei das erste Übertragungselement (174) und das zweite Übertragungselement (176) konzentrisch zur Längsachse (70) des Instrumententrägers (62) ausgerichtet und unabhängig voneinander antreibbar sind, wobei das zweite Übertragungselement (176) rohrartig gestaltet ist und das erste Übertragungselement (174) zumindest abschnittsweise umschließt.

3. Handhabungsvorrichtung (20) nach Anspruch 2, wobei die Handhabungsvorrichtung (20) ferner einen dritten Bewegungsfreiheitsgrad und einen vierten Bewegungsfreiheitsgrad für das aufgenommene Instrument (22) bereitstellt,
- wobei dem dritten Bewegungsfreiheitsgrad ein dritter Instrumentenantrieb (78), ein drittes Übertragungselement (178) und ein dritter Übertragungsanschluss (158) zugeordnet sind, die zur Drehübertragung miteinander gekoppelt sind, insbesondere durch Zahnradstufen,
- wobei dem vierten Bewegungsfreiheitsgrad ein vierter Instrumentenantrieb (80), ein viertes Übertragungselement (180) und ein vierter Übertragungsanschluss (160) zugeordnet sind, die zur Drehübertragung miteinander gekoppelt sind, insbesondere durch Zahnradstufen, und
- wobei das dritte Übertragungselement (178) und das vierte Übertragungselement (180) konzentrisch zur Längsachse (70) des Instrumententrägers (62) ausgerichtet und unabhängig voneinander antreibbar sind, wobei das vierte Übertragungselement (180) rohrartig gestaltet ist und das dritte Übertragungselement (178) zumindest abschnittsweise umschließt, wobei das dritte Übertragungselement (178) rohrartig gestaltet ist und das zweite Übertragungselement (176) zumindest abschnittsweise umschließt.

4. Handhabungsvorrichtung (20) nach einem der vorhergehenden Ansprüche, wobei das zumindest eine Übertragungselement (174, 176, 178, 180) zur Momentenübertragung an seinem proximalen Ende mit dem Instrumentenantrieb (74, 76, 78, 80) und an seinem distalen Ende mit dem Übertragungsanschluss (154, 156, 158, 160) gekoppelt ist, wobei bei einer Verdrehung des Instrumententrägers (62) um die Längsachse (70), die eine betragsgleiche und richtungsgleiche Schwenkbewegung des Übertragungsanschlusses (154, 156, 158, 160) bewirkt, der jeweils zugeordnete Instrumentenantrieb (74, 76, 78, 80) in seiner definierten Relativlage bezüglich des Gestells (30) verbleibt.

5. Handhabungsvorrichtung (20) nach einem der vorhergehenden Ansprüche, wobei am Gestell (30) zumindest eine Längsführung (38) ausgebildet ist, an der ein Schlitten (40) aufgenommen ist, der den zumindest einen Instrumententräger (62) trägt, wobei der zumindest eine Instrumententräger (62) gemeinsam mit dem Schlitten (40) relativ zum Gestell (30) verfahrbar ist.

6. Handhabungsvorrichtung (20) nach einem der vorhergehenden Ansprüche, wobei der zumindest eine Instrumentenantrieb (74, 76, 78, 80), vorzugsweise jeder Instrumentenantrieb, der dem Instrumententräger (62) zugeordnet ist, am Schlitten (40) aufgenommen ist, und wobei, sofern eine Mehrzahl von Instrumentenantrieben (74, 76, 78, 80) am Schlitten (40) aufgenommen ist, die Instrumentenantriebe (74, 76, 78, 80) umfänglich des Instrumententrägers (62), insbesondere umfänglich des Übertragungsabschnitts (126), und parallel zu diesem orientiert angeordnet sind, wobei die Instrumentenantriebe (74, 76, 78, 80) vorzugsweise um den Instrumententräger (62) versetzt zu diesem am Schlitten (40) angeordnet sind, wobei der Instrumententräger (62) vorzugsweise in einem Randbereich des Schlittens (40) angeordnet ist, und wobei vorzugsweise zumindest zwei Instrumentenantriebe (74, 76, 78, 80) auf gegenüberliegenden Seiten des Schlittens (40) aufgenommen sind.

7. Handhabungsvorrichtung (20) nach Anspruch 5 oder 6, wobei der Schlitten (40) mit einem Längsantrieb (50) gekoppelt ist, insbesondere mit einem Gewindetrieb oder Kugelgewindetrieb, wobei der Längsantrieb (50) vorzugsweise einen gestellfest aufgenommenen Motor (52) umfasst, der eine Spindel (54) antreibt und/oder wobei am Schlitten (40) ein Rotationsantrieb (224) aufgenommen ist, der eine Rotation des Instrumententrägers (62) um seine Längsachse (70) bewirkt, wobei die Rotation des Instrumententrägers (62) eine Drehmitnahme des zumindest einen Übertragungsanschlusses (154, 156, 158, 160) um die Längsachse (70) bewirkt.

8. Handhabungsvorrichtung (20) nach einem der vorhergehenden Ansprüche, wobei der Aufnahmeabschnitt (64) des zumindest einen Instrumententrägers (62) als Rastaufnahme (244) für ein proximales Ende eines Instruments (22), insbesondere eines Instruments für die minimal-invasive Chirurgie oder Diagnose, ausgebildet ist, und wobei am Aufnahmeabschnitt (64) vorzugsweise ein Drehlagesicherungselement (258) ausgebildet ist, das im eingerückten Zustand mit einem Gegenelement (264) eines Instrumentenarms (24) zusammenwirkt, um eine definierte Drehlage zwischen dem Instrumentenarm (24) und dem Instrumententräger (62) zu bewirken.

9. Handhabungsvorrichtung (20) nach einem der vorhergehenden Ansprüche, umfassend zumindest einen ersten Instrumententräger (62-1) und einen zweiten Instrumententräger (62-2), die beweglich am Gestell (30) aufgenommen und zumindest abschnittsweise relativ zueinander beweglich sind, wobei jeder Instrumententräger (62-1, 62-2) mit einem Aufnahmeabschnitt (64) zur Aufnahme eines Instrumentenarms (24) versehen ist, wobei die Instrumententräger (62-1, 62-2) parallel zueinander und einander benachbart am Gestell (30) aufgenommen sind, und wobei Längsachsen (70) des ersten Instrumententrägers (62-1) und des zweiten Instrumententrägers (62-2) vorzugsweise um ein Versatzmaß (a) voneinander versetzt sind, das ein Verhältnis zu einem Bauraumdurchmesser (D) der Instrumententräger (62) aufweist, das weniger als 3.5:1, vorzugsweise weniger als 2.5:1, weiter bevorzugt weniger als 1.5:1 beträgt.

10. Handhabungsvorrichtung (20) nach Anspruch 9, wobei der erste Instrumententräger (62-1) an einem ersten Schlitten (40-1) und der zweite Instrumententräger (62-2) an einem zweiten Schlitten (40-2) aufgenommen ist, wobei der erste Instrumententräger (62-1) und der zweite Instrumententräger (62-2) in einander zugewandten Randbereichen der Schlitten (40-2) angeordnet sind, insbesondere einander unmittelbar benachbart.

11. Handhabungsvorrichtung (20) nach einem der vorhergehenden Ansprüche, wobei zumindest ein Instrumentenantrieb (74, 76, 78, 80) mit einem positionsgeregelten Motor vorgesehen ist, der derart angesteuert ist, dass während der Verdrehung des Instrumententrägers (62) um die Längsachse (70) der mit dem Instrumentenantrieb (74, 76, 78, 80) gekoppelte Übertragungsanschluss (154, 156, 158, 160) eine lokale Ausgleichsbewegung um seine Achse ausführt, so dass eine relative Drehwinkellage des Übertragungsanschlusses (154, 156, 158, 160) in Bezug auf den Instrumententräger (62) beibehalten wird.

12. Handhabungsvorrichtung (20) nach einem der Ansprüche 1 bis 10, wobei zumindest ein Instrumentenantrieb (74, 76, 78, 80) mit einem rastmomentarmen Motor oder mit einer Kupplung vorgesehen ist, wobei abtriebsseitig des Übertragungselements (174, 176, 178, 180) Selbsthemmung auftritt, so dass während der Verdrehung des Instrumententrägers (62) um die Längsachse (70) der mit dem Instrumentenantrieb (74, 76, 78, 80) gekoppelte oder koppelbare Übertragungsanschluss (154, 156, 158, 160) seine relative Drehwinkellage in Bezug auf den Instrumententräger (62) beibehält.

13. Handhabungsvorrichtung (20) nach einem der vorhergehenden Ansprüche, wobei der zumindest eine Übertragungsanschluss (154, 156, 158, 160) ein Mitnahmeprofil (268) aufweist, das formschlüssig mit einem Gegenprofil (278) eines instrumentenseitigen Instrumenteneingangs (276) koppelbar ist, wobei das Mitnahmeprofil (268) eine Ausrichtkontur (272) zu Montagevereinfachung umfasst, wobei die Ausrichtkontur (272) vorzugsweise eine radiale und rotatorische Vorausrichtung bewirkt, wobei das Mitnahmeprofil (270) vorzugsweise eine Mitnahmeausnehmung (270) am distalen Ende des Übertragungsanschlusses (154, 156, 158, 160) umfasst, wobei in einem Stirnbereich des Mitnahmeprofils (268) geneigte Versatzflächen (274) angeordnet sind, die die Mitnahmeausnehmung (270) umgeben.

14. Handhabungsvorrichtung (20) nach einem der vorhergehenden Ansprüche, wobei die Antriebsschnittstelle (130) eine Mehrzahl von Übertragungsanschlüssen (154, 156, 158, 160) umfasst, die im Aufnahmeabschnitt (64) angeordnet sind, und wobei zumindest einige der Übertragungsanschlüsse (154, 156, 158, 160) axial voneinander versetzt sind, so dass instrumentenseitige Instrumenteneingänge (276), die den Übertragungsanschlüssen (154, 156, 158, 160) zugeordnet sind, beim Fügen des Instrumentenarms (24) nacheinander mit den Übertragungsanschlüssen (154, 156, 158, 160) gekoppelt werden und/oder wobei der Instrumentenantrieb (74, 76, 78, 80), der dem zumindest einen Übertragungsanschluss (154, 156, 158, 160) zugeordnet ist, beim Fügen des Instrumentenarms (24) den Übertragungsanschluss (154, 156, 158, 160) hin- und herschwenkend antreibt, um das Einrücken des Mitnahmeprofils (268) und des Gegenprofils (278) zu unterstützen, und wobei vorzugsweise eine Mehrzahl von Übertragungsanschlüssen (154, 156, 158, 160) durch die ihnen zugeordneten Instrumentenantriebe (74, 76, 78, 80) zumindest abschnittsweise gleichzeitig hin- und herschwenkend angetrieben werden, wobei der Antrieb bei verschiedenen Drehgeschwindigkeiten erfolgt.

15. Manipulationssystem für minimal-invasive chirurgische Operationen, insbesondere Telemanipulationssystem für minimal-invasive Single-Port-Operationen, mit einer Ansteuerungsplattform (18), die eine Handhabungsvorrichtung (20) nach einem der vorhergehenden Ansprüche aufweist.

## Claims

1. Manipulating apparatus (20) for holding and handling instruments (22) for minimally invasive procedures, comprising a frame (30) and at least one instrument carrier (62) including a holding section (64) for holding an instrument arm (24),
- wherein the instrument carrier (62) is movably mounted to the frame (30) and arranged, for movement thereof, to be coupled with at least one drive (50, 224), wherein the instrument carrier (62) is at least sectionally rotatable about its longitudinal axis (70) relative to the frame (30),
- wherein the holding section (64) comprises a driving interface (130) for transmitting mechanical energy to the instrument arm (24),
- wherein the driving interface (130) involves at least one transmission port (154, 156, 158, 160) for motion transmission, particularly for torque transmission,
- wherein an instrument drive (74, 76, 78, 80) is assigned to the transmission port (154, 156, 158, 160),
- wherein the instrument carrier (62), at least sectionally along its longitudinal extension, comprises a transmission section (126), in which at least one transmission element (174, 176, 178, 180) is arranged, which enables a motion transmission between the instrument drive (74, 76, 78, 80) and the transmission port (154, 156, 158, 160),
- wherein the at least one transmission element (174, 176, 178, 180) is oriented in a fashion concentrically with respect to the longitudinal axis (70) of the instrument carrier (62), and
- wherein the transmission port (154, 156, 158, 160) is arranged in an off-center fashion with respect to the longitudinal axis (70), so that the transmission port (154, 156, 158, 160) is pivoted during the rotation of the instrument carrier (62) about the longitudinal axis (70) thereof.

2. Manipulating apparatus (20) according to claim 1, wherein the driving interface (130) of the at least one instrument carrier (62) comprises at least two, preferably at least three or four, transmission ports (154, 156, 158, 160), at least two of which are arranged in a fashion radially displaced from the longitudinal axis (70) and spaced from one another, wherein at least two, preferably at least three or four, transmission elements (174, 176, 178, 180) are arranged in the transmission section (126), which are assigned to the at least two transmission ports (154, 156, 158, 160), wherein at least two, preferably three or four, instrument drives (74, 76, 78, 80) are provided,
- wherein the manipulating apparatus (20) provides a first degree of freedom of movement for a mounted instrument (22), to which a first instrument drive (74), a first transmission element (174) and a first transmission port (154) are assigned, which are coupled with one another for rotation transmission, particularly by gear stages,
- wherein the manipulating apparatus (20) provides a second degree of freedom of movement for a mounted instrument (22), to which a second instrument drive (76), a second transmission element (176) and a second transmission port (156) are assigned, which are coupled with one another for rotation transmission, particularly by gear stages, and
- wherein the first transmission element (174) and the second transmission element (176) are oriented in a fashion concentrically with respect to the longitudinal axis (70) of the instrument carrier (62) and drivable in a fashion independently of one another, wherein the second transmission element (176) is arranged in a tubular fashion and at least sectionally surrounds the first transmission element (174).

3. Manipulating apparatus (20) according to claim 2, wherein the manipulating apparatus (20) further provides a third degree of freedom of movement and a fourth degree of freedom of movement for the mounted instrument (22),
- wherein a third instrument drive (78), a third transmission element (178) and a third transmission port (158) are assigned to the third degree of freedom of movement, which are coupled with one another for rotation transmission, particularly by gear stages,
- wherein a fourth instrument drive (80), a fourth transmission element (180) and a fourth transmission port (160) are assigned to the fourth degree of freedom of movement, which are coupled with one another for rotation transmission, particularly by gear stages, and
- wherein the third transmission element (178) and the fourth transmission element (180) are oriented in a fashion concentrically with respect to the longitudinal axis (70) of the instrument carrier (62) and drivable in a fashion independently of one another, wherein the fourth transmission element (180) is arranged in a tubular fashion and at least sectionally surrounds the third transmission element (178), wherein the third transmission element (178) is arranged in a tubular fashion and at least sectionally surrounds the second transmission element (176).

4. Manipulating apparatus (20) according to any of the preceding claims, wherein the at least one transmission element (174, 176, 178, 180) is, for torque transmission, at its proximal end, coupled with the instrument drive (74, 76, 78, 80) and, at its distal end, with the transmission port (154, 156, 158, 160), wherein, when the instrument carrier (62) is rotated about the longitudinal axis (70), which effects a pivot movement of the transmission port (154, 156, 158, 160) of the same magnitude and in the same direction, the respectively assigned instrument drive (74, 76, 78, 80) remains in its defined relative position with respect to the frame (30).

5. Manipulating apparatus (20) according to any of the preceding claims, wherein at least one longitudinal guide (38) is formed at the frame (30), at which a carriage (40) is mounted, which supports the at least one instrument carrier (62), wherein the at least one instrument carrier (62) and the carriage (40) are jointly movable with respect to the frame (30).

6. Manipulating apparatus (20) according to any of the preceding claims, wherein the at least one instrument drive (74, 76, 78, 80), preferably each instrument drive, which is assigned to the instrument carrier (62) is mounted at the carriage (40), and wherein, provided that a plurality of instrument drives (74, 76, 78, 80) is mounted at the carriage (40), the instrument drives (74, 76, 78, 80) are arranged in the periphery of the instrument carrier (62), particularly in the periphery of the transmission sections (126), and in a fashion oriented in parallel with respect to the instrument carrier (62), wherein the instrument drives (74, 76, 78, 80) are preferably arranged in a fashion about and offset from the instrument carrier (62) at the carriage (40), wherein the instrument carrier (62) is preferably arranged in a boundary region of the carriage (40), and wherein preferably at least two instrument drives (74, 76, 78, 80) are arranged at opposite sides of the carriage (40).

7. Manipulating apparatus (20) according to claim 5 or 6, wherein the carriage (40) is coupled with a longitudinal drive (50), particularly with a screw drive or ball screw drive, wherein the longitudinal drive (50) preferably involves a motor (52) that is fixedly arranged with respect to the frame, wherein the motor (52) drives a spindle (54), and/or wherein a rotation drive (224) is mounted at the carriage (40), the rotation drive (224) effecting a rotation of the instrument carrier (62) about its longitudinal axis (70), wherein the rotation of the instrument carrier (62) effects a rotation entrainment of the at least one transmission port (154, 156, 158, 160) about the longitudinal axis (70).

8. Manipulating apparatus (20) according to any of the preceding claims, wherein the holding section (64) of the at least one instrument carrier (62) is arranged as a locking receptacle (244) for a proximal end of an instrument (22), particularly an instrument for minimally invasive surgery or diagnosis, and wherein at the holding section (64) preferably a rotational position securing element (258) is formed, which cooperates in the engaged state with a counter element (264) of an instrument arm (24) so as to effect a defined rotation position between the instrument arm (24) and the instrument carrier (62).

9. Manipulating apparatus (20) according to any of the preceding claims, comprising at least a first instrument carrier (62-1) and a second instrument carrier (62-2), which are movably mounted at the frame (30) and at least sectionally movable with respect to one another, wherein each instrument carrier (62-1, 62-2) is provided with a holding section (64) for holding an instrument arm (24), wherein the instrument carriers (62-1, 62-2) are mounted at the frame (30) in a fashion parallel to one another and adjacent to one another, and preferably wherein longitudinal axes (70) of the first instrument carrier (62-1) and the second instrument carrier (62-2) are spaced from one another at an offset dimension (a) having a ratio with an installation space diameter (D) of the instrument carrier (62), which is less than 3.5:1, preferably less than 2.5:1, further preferred less than 1.5:1.

10. Manipulating apparatus (20) according to claim 9, wherein the first instrument carrier (62-1) is mounted at the first carriage (40-1) and the second instrument carrier (62-2) is mounted at the second carriage (40-2), wherein the first instrument carrier (62-1) and the second instrument carrier (62-2) are arranged in facing boundary regions of the carriages (40-1, 40-2), particularly in a fashion directly adjacent to one another.

11. Manipulating apparatus (20) according to any of the preceding claims, wherein at least one instrument drive (74, 76, 78, 80) is provided with a position-controlled motor, which is controlled in such a way that, when the instrument carrier (62) is rotated about the longitudinal axis (70), the transmission port (154, 156, 158, 160), which is coupled with the instrument drive (74, 76, 78, 80) performs a local compensation movement about its axis in such a way that a relative rotation angle position of the transmission port (154, 156, 158, 160) with respect to the instrument carrier (62) is maintained.

12. Manipulating apparatus (20) according to any of the claims 1 to 10, wherein at least one instrument drive (74, 76, 78, 80) is provided, involving a low-detent-torque motor or a clutch, wherein self-locking is present at the output-side of the transmission element (174, 176, 178, 180) in such a way that, when the instrument carrier (62) is rotated about the longitudinal axis (70), the transmission port (154, 156, 158, 160) that is coupled or arranged to be coupled with the instrument drive (74, 76, 78, 80) maintains its relative rotation angle position with respect to the instrument carrier (62).

13. Manipulating apparatus (20) according to any of the preceding claims, wherein the at least one transmission port (154, 156, 158, 160) comprises a driving profile (268), which is arranged to be coupled in a positive-locking fashion with a mating profile (278) of an instrument-side instrument input (276), wherein the driving profile (268) comprises an orientation contour (272) for facilitating assembly, wherein the orientation contour (272) preferably effects a radial and rotatory pre-orientation, wherein the driving profile (270) preferably comprises a driving recess (270) at the distal end of the transmission port (154, 156, 158, 160), and wherein, in a frontal region of the driving profile (268), inclined offset surfaces (274) are arranged, which surround the driving recess (270).

14. Manipulating apparatus (20) according to any of the preceding claims, wherein the driving interface (130) comprises a plurality of transmission ports (154, 156, 158, 160), which are arranged in the holding section (64), and wherein at least some of the transmission ports (154, 156, 158, 160) are axially offset from one another so that instrument-sided instrument inputs (276), which are assigned to the transmission ports (154, 156, 158, 160) are successively coupled with the transmission ports (154, 156, 158, 160) when the instrument arm (24) is attached, and/or wherein the instrument drive (74, 76, 78, 80) that is assigned to the at least one transmission port (154, 156, 158, 160) drives the transmission port (154, 156, 158, 160) in a reciprocating pivoting movement when the instrument arm (24) is attached so as to support the engagement of the driving profile (268) and the mating profile (278), and wherein preferably a plurality of transmission ports (154, 156, 158, 160) is simultaneously driven in a reciprocating pivoting movement by the instrument drives (74, 76, 78, 80) respectively assigned thereto, wherein the actuation involves different rotation speeds.

15. Manipulating system for minimally invasive surgical operations, particularly remote manipulating system for minimally invasive single-port-operations, comprising a control platform (18) that comprises a manipulating apparatus (20) as claimed in any of the preceding claims.

## Revendications

1. Dispositif de manipulation (20) servant à la réception et à la manipulation d'instruments (22) pour des interventions minimalement invasives, comportant un cadre (30) et au moins un support d'instrument (62) doté d'une partie de réception (64) servant à la réception d'un bras d'instrument (24),
- le support d'instrument (62) étant reçu de manière mobile sur le cadre (30) et pouvant être accouplé à au moins un entraînement (50, 224) pour son mouvement, le support d'instrument (62) pouvant tourner au moins dans certaines parties autour de son axe longitudinal (70) relativement au cadre (30),
- la partie de réception (64) comprenant une interface d'entraînement (130) servant à la transmission d'énergie mécanique au bras d'instrument (24),
- l'interface d'entraînement (130) comportant au moins un raccord de transmission (154, 156, 158, 160) pour la transmission de mouvement, en particulier pour la transmission de couple,
- un entraînement d'instrument (74, 76, 78, 80) étant associé au raccord de transmission (154, 156, 158, 160),
- le support d'instrument (62) comprenant, au moins dans certaines parties le long de son étendue longitudinale, une partie de transmission (126) dans laquelle au moins un élément de transmission (174, 176, 178, 180) est disposé, lequel permet une transmission de mouvement entre l'entraînement d'instrument (74, 76, 78, 80) et le raccord de transmission (154, 156, 158, 160),
- l'au moins un élément de transmission (174, 176, 178, 180) étant orienté concentriquement à l'axe longitudinal (70) du support d'instrument (62), et
- le raccord de transmission (154, 156, 158, 160) étant disposé de manière excentrée par rapport à l'axe longitudinal (70), de sorte que le raccord de transmission (154, 156, 158, 160) soit pivoté autour de l'axe longitudinal (70) du support d'instrument (62) pendant la rotation de ce dernier.

2. Dispositif de manipulation (20) selon la revendication 1, l'interface d'entraînement (130) de l'au moins un support d'instrument (62) comprenant au moins deux, de préférence au moins trois ou quatre, raccords de transmission (154, 156, 158, 160), parmi lesquels au moins deux sont disposés de manières décalée radialement de l'axe longitudinal (70) et de manière espacée les uns des autres, au moins deux, de préférence au moins trois ou quatre, éléments de transmission (174, 176, 178, 180) étant disposés dans la partie de transmission (126), lesquels sont associés aux au moins deux raccords de transmission (154, 156, 158, 160), au moins deux, de préférence au moins trois ou quatre, entraînements d'instrument (74, 76, 78, 80) étant prévus,
- le dispositif de manipulation (20) fournissant un premier degré de liberté de mouvement pour un instrument reçu (22), premier degré de liberté auquel sont associés un premier entraînement d'instrument (74), un premier élément de transmission (174) et un premier raccord de transmission (154), qui sont accouplés les uns aux autres pour la transmission de rotation, en particulier par le biais d'étages de roues dentées,
- le dispositif de manipulation (20) fournissant un deuxième degré de liberté de mouvement pour un instrument reçu (22), deuxième degré de liberté auquel sont associés un deuxième entraînement d'instrument (76), un deuxième élément de transmission (176) et un deuxième raccord de transmission (156), qui sont accouplés les uns aux autres pour la transmission de rotation, en particulier par le biais d'étages de roues dentées, et
- le premier élément de transmission (174) et le deuxième élément de transmission (176) étant orientés concentriquement à l'axe longitudinal (70) du support d'instrument (62) et pouvant être entraînés indépendamment l'un de l'autre, le deuxième élément de transmission (176) étant configuré de manière tubulaire et entourant le premier élément de transmission (174) au moins dans certaines parties.

3. Dispositif de manipulation (20) selon la revendication 2, le dispositif de manipulation (20) fournissant en outre un troisième degré de liberté de mouvement et un quatrième degré de liberté de mouvement pour l'instrument reçu (22),
- un troisième entraînement d'instrument (78), un troisième élément de transmission (178) et un troisième raccord de transmission (158) étant associés au troisième degré de liberté de mouvement, lesquels sont accouplés les uns aux autres pour la transmission de rotation, en particulier par le biais d'étages de roues dentées,
- un quatrième entraînement d'instrument (80), un quatrième élément de transmission (180) et un quatrième raccord de transmission (160) étant associés au quatrième degré de liberté de mouvement, lesquels sont accouplés les uns aux autres pour la transmission de rotation, en particulier par le biais d'étages de roues dentées, et
- le troisième élément de transmission (178) et le quatrième élément de transmission (180) étant orientés concentriquement à l'axe longitudinal (70) du support d'instrument (62) et pouvant être entraînés indépendamment l'un de l'autre, le quatrième élément de transmission (180) étant configuré de manière tubulaire et entourant le troisième élément de transmission (178) au moins dans certaines parties, le troisième élément de transmission (178) étant configuré de manière tubulaire et entourant le deuxième élément de transmission (176) au moins dans certaines parties.

4. Dispositif de manipulation (20) selon l'une des revendications précédentes, l'au moins un élément de transmission (174, 176, 178, 180) étant accouplé, à son extrémité proximale, à l'entraînement d'instrument (74, 76, 78, 80) et, à son extrémité distale, au raccord de transmission (154, 156, 158, 160) pour la transmission de couple, et lors d'une rotation du support d'instrument (62) autour de l'axe longitudinal (70), qui provoque un mouvement de pivotement de même amplitude et de même sens du raccord de transmission (154, 156, 158, 160), l'entraînement d'instrument (74, 76, 78, 80) respectivement associé demeurant dans sa position relative définie par rapport au cadre (30).

5. Dispositif de manipulation (20) selon l'une des revendications précédentes, au moins un guide longitudinal (38) étant réalisé sur le cadre (30), guide sur lequel un chariot (40) est reçu, lequel supporte l'au moins un support d'instrument (62), l'au moins un support d'instrument (62) étant déplaçable conjointement avec le chariot (40) relativement au cadre (30).

6. Dispositif de manipulation (20) selon l'une des revendications précédentes, l'au moins un entraînement d'instrument (74, 76, 78, 80), de préférence chaque entraînement d'instrument, qui est associé au support d'instrument (62) étant reçu sur le chariot (40), et si une pluralité d'entraînements d'instrument (74, 76, 78, 80) sont reçus sur le chariot (40), les entraînements d'instrument (74, 76, 78, 80) sont disposés à la périphérie du support d'instrument (62), en particulier à la périphérie de la partie de transmission (126), et de manière orientée parallèlement à celui-ci, les entraînements d'instrument (74, 76, 78, 80) étant disposés de préférence autour du support d'instrument (62) et de manière décalée par rapport à celui-ci sur le chariot (40), le support d'instrument (62) étant disposé de préférence dans une région de bord du chariot (40), et de préférence au moins deux entraînements d'instrument (74, 76, 78, 80) étant reçus sur des côtés opposés du chariot (40).

7. Dispositif de manipulation (20) selon la revendication 5 ou 6, le chariot (40) étant accouplé à un entraînement longitudinal (50), en particulier à une vis d'entraînement ou une vis d'entraînement à billes, l'entraînement longitudinal (50) comportant de préférence un moteur (52) reçu de manière solidaire du cadre, lequel moteur entraîne une broche (54) et/ou un entraînement rotatif (224) étant reçu sur le chariot (40), lequel entraînement rotatif provoque une rotation du support d'instrument (62) autour de son axe longitudinal (70), la rotation du support d'instrument (62) provoquant un entraînement en rotation de l'au moins raccord de transmission (154, 156, 158, 160) autour de l'axe longitudinal (70).

8. Dispositif de manipulation (20) selon l'une des revendications précédentes, la partie de réception (64) de l'au moins un support d'instrument (62) étant réalisée comme logement d'encliquetage (244) pour une extrémité proximale d'un instrument (22), en particulier d'un instrument pour la chirurgie minimalement invasive ou le diagnostic minimalement invasif, et de préférence un élément de fixation de position en rotation (258) étant réalisé sur la partie de réception (64), lequel coopère à l'état enclenché avec un élément conjugué (264) d'un bras d'instrument (24), afin de provoquer une position en rotation définie du bras d'instrument (24) et du support d'instrument (62).

9. Dispositif de manipulation (20) selon l'une des revendications précédentes, comportant au moins un premier support d'instrument (62-1) et un deuxième support d'instrument (62-2), qui sont reçus de manière mobile sur le cadre (30) et sont mobiles l'un par rapport à l'autre au moins dans certaines parties, chaque support d'instrument (62-1, 62-2) étant doté d'une partie de réception (64) servant à la réception d'un bras d'instrument (24), les supports d'instrument (62-1, 62-2) étant reçus parallèlement l'un à l'autre et de manière adjacente l'un à l'autre sur le cadre (30),
et les axes longitudinaux (70) du premier support d'instrument (62-1) et du deuxième support d'instrument (62-2) étant décalés l'un par rapport à l'autre de préférence d'une quantité de décalage (a) qui présente un rapport à un diamètre d'espace d'installation (D) du support d'instrument (62) qui vaut moins de 3,5:1, de préférence moins de 2,5:1, de manière particulièrement préférée moins de 1,5:1.

10. Dispositif de manipulation (20) selon la revendication 9, le premier support d'instrument (62-1) étant reçu sur un premier chariot (40-1) et le deuxième support d'instrument (62-2) étant reçu sur un deuxième chariot (40-2), le premier support d'instrument (62-1) et le deuxième support instrument (62-2) étant disposés dans des régions de bord, tournées l'une vers l'autre, des chariots (40-2), en particulier de manière immédiatement adjacente l'un à l'autre.

11. Dispositif de manipulation (20) selon l'une des revendications précédentes, au moins un entraînement d'instrument (74, 76, 78, 80) étant doté d'un moteur à réglage de position qui est commandé de telle sorte que pendant la rotation du support d'instrument (62) autour de l'axe longitudinal (70), le raccord de transmission (154, 156, 158, 160) accouplé à l'entraînement d'instrument (74, 76, 78, 80) effectue un mouvement de compensation local autour de son axe, de sorte qu'une position angulaire de rotation relative du raccord de transmission (154, 156, 158, 160) par rapport au support d'instrument (62) soit maintenue.

12. Dispositif de manipulation (20) selon l'une des revendications 1 à 10, au moins un entraînement d'instrument (74, 76, 78, 80) étant doté d'un moteur à faible couple de détente ou d'un embrayage, un autoblocage se produisant du côté de la sortie de l'élément de transmission (174, 176, 178, 180), de telle sorte que pendant la rotation du support d'instrument (62) autour de l'axe longitudinal (70), le raccord de transmission (154, 156, 158, 160) accouplé ou pouvant être accouplé à l'entraînement d'instrument (74, 76, 78, 80) maintienne sa position angulaire en rotation relative par rapport au support d'instrument (62).

13. Dispositif de manipulation (20) selon l'une des revendications précédentes, l'au moins un raccord de transmission (154, 156, 158, 160) comprenant un profil d'entraînement (268) qui peut être accouplé avec complémentarité de forme à un profil conjugué (278) d'une entrée d'instrument (276) côté instrument, le profil d'entraînement (268) comportant un contour d'orientation (272) servant à simplifier le montage, le contour d'orientation (272) produisant de préférence une direction préalable radiale et de rotation, le profil d'entraînement (270) comportant de préférence un évidement d'entraînement (270) à l'extrémité distale du raccord de transmission (154, 156, 158, 160), des surfaces de décalage inclinées (274) étant disposées dans une région frontale du profil d'entraînement (268), lesquelles surfaces entourent l'évidement d'entraînement (270).

14. Dispositif de manipulation (20) selon l'une des revendications précédentes, l'interface d'entraînement (130) comportant une pluralité de raccords de transmission (154, 156, 158, 160) qui sont disposés dans la partie de réception (64), et au moins certains raccords de transmission (154, 156, 158, 160) étant décalés axialement les uns par rapport aux autres, de telle sorte que des entrées d'instrument (276) côté instrument, qui sont associées aux raccords de transmission (154, 156, 158, 160), soient accouplées tour à tour aux raccords de transmission (154, 156, 158, 160) lors de l'assemblage du bras d'instrument (24) et/ou l'entraînement d'instrument (74, 76, 78, 80), qui est associé à l'au moins un raccord de transmission (154, 156, 158, 160), entraînant le raccord de transmission (154, 156, 158, 160) de manière pivotante suivant un mouvement de va-et-vient lors de l'assemblage du bras d'instrument (24), afin de favoriser l'enclenchement du profil d'entraînement (268) et du profil conjugué (278), et de préférence une pluralité de raccords de transmission (154, 156, 158, 160) étant entraînés simultanément de manière pivotante suivant un mouvement de va-et-vient au moins dans certaines parties au moyen des entraînements d'instrument (74, 76, 78, 80) qui sont associés à ceux-ci, l'entraînement s'effectuant à différentes vitesses de rotation.

15. Système de manipulation pour des opérations chirurgicales minimalement invasives, en particulier système de télémanipulation pour des opérations à port unique minimalement invasives, comportant une plate-forme de commande (18) qui comprend un dispositif de manipulation (20) selon l'une des revendications précédentes.
